(19) Europäisches Patentamt
European Patent Office
Office européen des brevets

(11) Veröffentlichungsnummer: **0 484 776 A1**

# (12) EUROPÄISCHE PATENTANMELDUNG

(21) Anmeldenummer: **91118315.0**

(22) Anmeldetag: **28.10.91**

(51) Int. Cl.[5]: **C07D 263/38**, A01N 43/76, C07D 413/04, C07D 233/70, A01N 43/50, C07D 263/46, C07C 271/28, C07C 275/28, C07C 323/63, C07C 275/34

(30) Priorität: **08.11.90 DE 4035437**

(43) Veröffentlichungstag der Anmeldung: **13.05.92 Patentblatt 92/20**

(84) Benannte Vertragsstaaten: **BE CH DE FR GB IT LI NL**

(71) Anmelder: **BAYER AG**
**W-5090 Leverkusen 1 Bayerwerk(DE)**

(72) Erfinder: **Ooms, Pieter, Dr.**
**Doerperhofstrasse 16**
**W-4150 Krefeld 1(DE)**
Erfinder: **Lürssen, Klaus, Dr.**
**August-Kierspel-Strasse 145**
**W-5060 Bergisch Gladbach 2(DE)**
Erfinder: **Santel, Hans-Joachim, Dr.**
**Grünstrasse 9a**
**W-5090 Leverkusen 1(DE)**
Erfinder: **Schmidt, Robert R., Dr.**
**Im Waldwinkel 110**
**W-5060 Bergisch Gladbach 2(DE)**

(54) **Arylsubstituierte Alkylidenstickstoffheterocyclen.**

(57) Neue arylsubstituierte Alkylidenstickstoffheterocyclen der allgemeinen Formel (I),

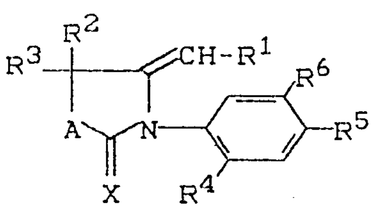

(I)

EP 0 484 776 A1

Verfahren zur ihrer Herstellung und ihre Verwendung als Herbizide sowie neuartige Zwischenprodukte der Formeln

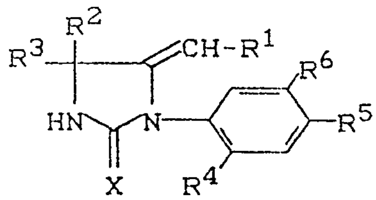

(Ia)

und

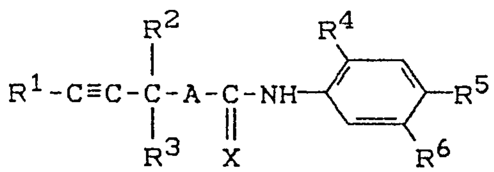

(IV)

Die Erfindung betrifft neue arylsubstituierte Alkyliden-Stickstoffheterocyclen, mehrere Verfahren zu ihrer Herstellung sowie ihre Verwendung als Herbizide.

Es ist bekannt, daß bestimmte arylsubstituierte Stickstoffheterocyclen, wie beispielsweise die Verbindung 1-(2-Fluor-5-isopropoxy-4-methylphenyl)-3-methyl-imidazolidin-2,5-dion herbizide Eigenschaften besitzen (vergleiche z. B. EP 363 585).

Die herbizide Wirksamkeit dieser vorbekannten Verbindungen gegenüber Problemunkräutern ist jedoch ebenso wie ihre Verträglichkeit gegenüber wichtigen Kulturpflanzen nicht in allen Anwendungsgebieten völlig zufriedenstellend.

Es wurden neue arylsubstituierte Alkylidenstickstoffheterocyclen der allgemeinen Formel (I),

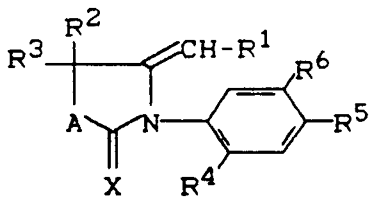

(I)

in welcher

| | |
|---|---|
| $R^1$ | für Wasserstoff, Halogen, Alkyl oder Aryl steht, |
| $R^2$ und $R^3$ | unabhängig voneinander jeweils für Wasserstoff, Alkyl oder Cycloalkyl stehen oder gemeinsam für einen zweifach verknüpften Alkandiylrest stehen, |
| $R^4$ | für Wasserstoff oder Halogen steht, |
| $R^5$ | für Wasserstoff, Halogen, Cyano oder Alkyl steht und |
| $R^6$ | für Halogenalkyl oder für einen Rest $-X^1-R^7$ steht oder |
| $R^5$ und $R^6$ | gemeinsam für einen zweifach verknüpften Rest der Formel |

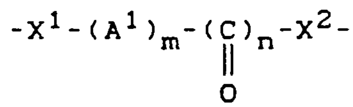

| | |
|---|---|
| | stehen, |
| A | für Sauerstoff oder für einen Rest $>N-R^8$ steht und |
| X | für Sauerstoff oder Schwefel steht, wobei |
| $R^7$ | für Wasserstoff, Alkyl, Alkenyl, Alkinyl, Cyanalkyl, Alkoxyalkyl, Alkoxyalkoxyalkyl, Alkoxyalkoxyalkoxyalkyl, Alkoxycarbonylalkyl, Heterocyclylalkyl oder Heterocyclyl steht, |
| $R^8$ | für Wasserstoff, Alkyl oder Halogenalkyl steht, |
| $A^1$ | für zweifach verknüpftes Alkandiyl steht, |
| $X^1$ | für Sauerstoff oder Schwefel steht, |
| $X^2$ | für Sauerstoff, Schwefel oder $>N-R^7$ steht, |
| n | für eine Zahl 0 oder 1 steht und |
| m | für eine Zahl 0 oder 1 steht, |

gefunden.

Weiterhin wurde gefunden, daß man die neuen arylsubstituierten Alkylidenstickstoffheterocyclen der allgemeinen Formel (I),

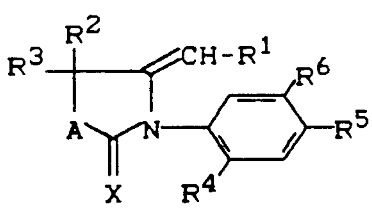

(I)

in welcher

$R^1$ für Wasserstoff, Halogen, Alkyl oder Aryl steht,

$R^2$ und $R^3$ unabhängig voneinander jeweils für Wasserstoff, Alkyl oder Cycloalkyl stehen oder gemeinsam für einen zweifach verknüpften Alkandiylrest stehen,

$R^4$ für Wasserstoff oder Halogen steht,

$R^5$ für Wasserstoff, Halogen, Cyano oder Alkyl steht und

$R^6$ für Halogenalkyl oder für einen Rest $-X^1-R^7$ steht oder

$R^5$ und $R^6$ gemeinsam für einen zweifach verknüpften Rest der Formel

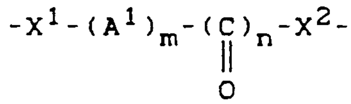

stehen,

A für Sauerstoff oder für einen Rest $>N-R^8$ steht und

X für Sauerstoff oder Schwefel steht, wobei

$R^7$ für Wasserstoff, Alkyl, Alkenyl, Alkinyl, Cyanalkyl, Alkoxyalkyl, Alkoxyalkoxyalkyl, Alkoxyalkoxyalkoxyalkyl, Alkoxycarbonylalkyl, Heterocyclylalkyl oder Heterocyclyl steht,

$R^8$ für Wasserstoff, Alkyl oder Halogenalkyl steht,

$A^1$ für zweifach verknüpftes Alkandiyl steht,

$X^1$ für Sauerstoff oder Schwefel steht,

$X^2$ für Sauerstoff, Schwefel oder $>N-R^7$ steht,

n für eine Zahl 0 oder 1 steht und

m für eine Zahl 0 oder 1 steht,

erhält, wenn man

(a) Alkinylverbindungen der Formel (II),

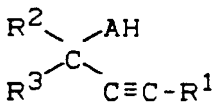

(II)

in welcher

$R^1$, $R^2$, $R^3$ und A die oben angegebene Bedeutung haben,

mit Iso(thio)cyanaten der Formel (III),

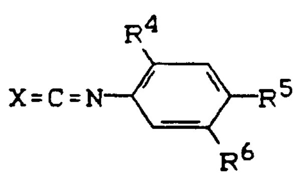

(III)

in welcher

$R^4$, $R^5$, $R^6$ und X die oben angegebene Bedeutung haben,

gegebenenfalls in Gegenwart eines Verdünnungsmittels und gegebenenfalls in Gegenwart eines Reaktionshilfsmittels umsetzt und anschließend die so erhältlichen Alkinylcarbamate (oder -harnstoffe) der Formel (IV),

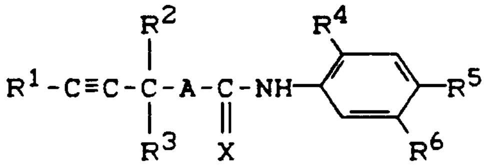

(IV)

in welcher

$R^1$, $R^2$, $R^3$, $R^4$, $R^5$, $R^6$, A und X die oben angegebene Bedeutung haben,

mit organischen Basen gegebenenfalls in Gegenwart eines Verdünnungsmittels cyclisiert; oder wenn man

(b) Alkinylverbindungen der Formel (IIa),

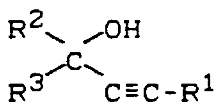

(IIa)

in welcher

$R^1$, $R^2$ und $R^3$ die oben angegebene Bedeutung haben,

mit Anilinderivaten der Formel (V),

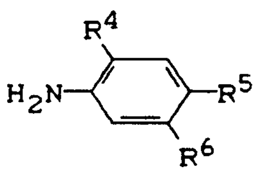

(V)

in welcher

$R^4$, $R^5$ und $R^6$ die oben angegebene Bedeutung haben,

in Gegenwart von Kohlendioxid sowie gegebenenfalls in Gegenwart eines Verdünnungsmittels und gegebenenfalls in Gegenwart eines Reaktionshilfsmittels umsetzt; oder wenn man

(c) die mit Hilfe des erfindungsgemäßen Verfahrens (a) erhältlichen arylsubstituierten Alkylidenstickstoffheterocyclen der Formel (Ia),

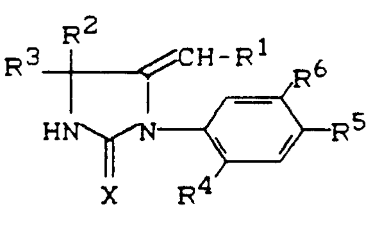

(Ia)

in welcher

$R^1$, $R^2$, $R^3$, $R^4$, $R^5$, $R^6$ und X die oben angegebene Bedeutung haben,

mit Alkylierungsmitteln der Formel (VI),

$R^{8-1}$-E (VI)

in welcher

$R^{8-1}$ für Alkyl oder Halogenalkyl steht und

E für eine elektronenanziehende Abgangsgruppe steht,

gegebenenfalls in Gegenwart eines Verdünnungsmittels und gegebenenfalls in Gegenwart eines Reaktionshilfsmittels umsetzt.

Schließlich wurde gefunden, daß die neuen arylsubstituierten Alkylidenstickstoffheterocyclen der allgemeinen Formel (I) herbizide, insbesondere auch selektiv herbizide Eigenschaften besitzen.

Überraschenderweise zeigen die erfindungsgemäßen arylsubstituierten Alkylidenstickstoffheterocyclen der allgemeinen Formel (I) bei vergleichbar guter Nutzpflanzenselektivität eine erheblich höhere herbizide Wirkung, als die aus dem Stand der Technik bekannten arylsubstituierten Stickstoffheterocyclen, wie beispielsweise die Verbindung 1-(2-Fluor-5-isopropoxy-4-methylphenyl)-3-methylimidazolin-2,5-dion. Letztere sind sowohl chemisch als auch wirkungsmäßig naheliegende Verbindungen.

Die erfindungsgemäßen arylsubstituierten Alkyliden-Stickstoffheterocyclen sind durch die Formel (I) allgemein definiert. Bevorzugt sind Verbindungen der Formel (I), bei welchen

$R^1$ für Wasserstoff, Fluor, Chlor, Brom, für geradkettiges oder verzweigtes Alkyl mit 1 bis 8 Kohlenstoffatomen oder für Phenyl steht,

$R^2$ und $R^3$ entweder unabhängig voneinander jeweils für Wasserstoff, für geradkettiges oder verzweigtes Alkyl mit 1 bis 8 Kohlenstoffatomen oder für Cycloalkyl mit 3 bis 7 Kohlenstoffatomen stehen oder gemeinsam für einen zweifach verknüpften geradkettigen oder verzweigten Alkandiylrest mit 2 bis 10 Kohlenstoffatomen stehen,

$R^4$ für Wasserstoff, Fluor, Chlor oder Brom steht,

$R^5$ für Wasserstoff, Fluor, Chlor, Brom, Cyano oder für geradkettiges oder verzweigtes Alkyl mit 1 bis 8 Kohlenstoffatomen steht und

$R^6$ für geradkettiges oder verzweigtes Halogenalkyl mit 1 bis 6 Kohlenstoffatomen und 1 bis 13 gleichen oder verschiedenen Halogenatomen oder für einen Rest $-X^1-R^7$ steht oder

$R^5$ und $R^6$ gemeinsam für einen zweifach verknüpften Rest der Formel

$$-X^1-(A^1)_m-(\underset{\underset{O}{\|}}{C})_n-X^2-$$

stehen,

A für Sauerstoff oder für einen Rest $>N-R^8$ steht und

X für Sauerstoff oder Schwefel steht, wobei

$R^7$ für Wasserstoff, für jeweils geradkettiges oder verzweigtes Alkyl mit 1 bis 12 Kohlenstoffatomen, Alkenyl mit 2 bis 12 Kohlenstoffatomen, Alkinyl mit 2 bis 12 Kohlenstoffatomen, Cyanalkyl mit 2 bis 12 Kohlenstoffatomen, Alkoxyalkyl, Alkoxyalkoxyalkyl, Alkoxyalkoxyalkoxyalkyl oder Alkoxycarbonylalkyl mit jeweils 1 bis 8 Kohlenstoffatomen in den einzelnen Alkoxy- und Alkylteilen, für Heterocyclylalkyl mit 1 bis 4 Kohlenstoffatomen im geradkettigen oder verzweigten Alkylteil oder für Heterocyclyl steht, wobei als Heterocyclylrest jeweils ein 3- bis 7-gliedriger gegebenenfalls auch benzannellierter oder mehrkernig kondensierter, gesättigter oder ungesättigter Heterocyclus mit 1 bis 5 Heteroatomen - insbesondere Stickstoff, Sauerstoff und/oder Schwefel- und 2 bis 10 Kohlenstoffatomen infrage kommt,

$R^8$ für Wasserstoff, für geradkettiges oder verzweigtes Alkyl mit 1 bis 8 Kohlenstoffatomen oder für geradkettiges oder verzweigtes Halogenalkyl mit 1 bis 4 Kohlenstoffatomen und 1 bis 9 gleichen oder verschiedenen Halogenatomen steht,

$A^1$ für geradkettiges oder verzweigtes zweifach verknüpftes Alkandiyl mit 1 bis 6 Kohlenstoffatomen steht,

$X^1$ für Sauerstoff oder Schwefel steht,

$X^2$ für Sauerstoff, Schwefel oder $>N-R^7$ steht,

n für eine Zahl 0 oder 1 steht und

m für eine Zahl 0 oder 1 steht.

Besonders bevorzugt sind Verbindungen der Formel (I), bei welchen

$R^1$ für Wasserstoff, Fluor, Chlor, Brom, Methyl, Ethyl, n- oder i-Propyl, n-, i-, s- oder t-Butyl oder für Phenyl steht,

$R^2$ und $R^3$ entweder unabhängig voneinander jeweils für Wasserstoff, Methyl, Ethyl, n- oder i-Propyl, n-, i-, s- oder t-Butyl, für Cyclopropyl, Cyclopentyl oder Cyclohexyl stehen oder gemeinsam für einen jeweils zweifach verknüpften 1,2-Ethandiylrest, 1,3-Propandiylrest, 1,4-Butandiylrest oder 1,5-Pentandiylrest stehen,

$R^4$ für Wasserstoff, Fluor oder Chlor steht,

$R^5$ für Wasserstoff, Fluor, Chlor, Cyano, Methyl, Ethyl, n- oder i-Propyl, n-, i-, s- oder t-Butyl steht und

$R^6$ für geradkettiges oder verzweigtes Halogenalkyl mit 1 bis 4 Kohlenstoffatomen und 1 bis 9 gleichen oder verschiedenen Halogenatomen oder für einen Rest $-X^1-R^7$ steht oder

$R^5$ und $R^6$ gemeinsam für einen zweifach verknüpften Rest der Formel

$$-X^1-(A^1)_m-(\underset{\underset{O}{\|}}{C})_n-X^2-$$

stehen,

A für Sauerstoff oder für einen Rest $>N-R^8$ steht und

X für Sauerstoff oder Schwefel steht, wobei

$R^7$ für Wasserstoff, Methyl, Ethyl, n- oder i-Propyl, n-, i-, s- oder t-Butyl, für Allyl, n- oder i-Butenyl, n- oder i-Pentenyl, n- oder i-Hexenyl, Propargyl, n- oder i-Butinyl, n- oder i-Pentinyl, n- oder i-Hexinyl, für Cyanmethyl, Cyanethyl, Cyanpropyl, für Methoxymethyl, Methoxyethyl, Methoxypropyl, Ethoxymethyl, Ethoxyethyl, Ethoxypropyl, Propoxymethyl, Propoxyethyl, Propoxypropyl, für Methoxyethoxyethyl, für Ethoxyethoxyethyl, für Propoxyethoxyethyl, für Methoxycarbonylmethyl, Ethoxycarbonylmethyl, Propoxycarbonylmethyl, Methoxycarbonylethyl, Ethoxycarbonylethyl, Propoxycarbonylethyl, Methoxycarbonylpropyl, Ethoxycarbonylpropyl, Propoxycarbonylpropyl, für Heterocyclylmethyl, Heterocyclylethyl oder Heterocyclyl steht, wobei als Heterocyclylrest ein fünfgliedriger oder 6-gliedriger, gesättigter oder ungesättigter Heterocyclus mit 1 bis 3 Heteroatomen, insbesondere Stickstoff, Sauerstoff und/oder Schwefel und 2 bis 5 Kohlenstoffatomen infrage kommt,

$R^8$ für Wasserstoff, Methyl, Ethyl, n- oder i-Propyl, n-, i-, s- oder t-Butyl oder für Halogenalkyl mit 1 bis 2 Kohlenstoffatomen und 1 bis 5 gleichen oder verschiedenen Halogenatomen steht,

$A^1$ für geradkettiges oder verzweigtes, zweifach verknüpftes Alkandiyl mit 1 bis 4 Kohlenstoffatomen steht,

$X^1$ für Sauerstoff oder Schwefel steht,

$X^2$ für Sauerstoff, Schwefel oder $>N-R^7$ steht,

n für eine Zahl 0 oder 1 steht und

m für eine Zahl 0 oder 1 steht.

Ganz besonders bevorzugt sind Verbindungen der Formel (I), bei welchen

$R^1$ für Wasserstoff, Chlor, Methyl, Ethyl, n- oder i-Propyl oder Phenyl steht,

$R^2$ und $R^3$ entweder unabhängig voneinander jeweils für Wasserstoff, Methyl, Ethyl, n- oder i-Propyl, oder Cyclopropyl, Cyclopentyl oder Cyclohexyl stehen, oder gemeinsam für einen 1,2-Ethandiylrest, einen 1,3-Propandiylrest, einen 1,4-Butandiylrest oder einen 1,5-Pentandiylrest stehen,

$R^4$ für Wasserstoff, Fluor oder Chlor steht

$R^5$ für Wasserstoff, Chlor, Brom, Cyano oder Methyl steht und

$R^6$ für Trifluormethyl oder für einen Rest $-X^1-R^7$ steht oder

$R^5$ und $R^6$ gemeinsam für einen zweifach verknüpften Rest der Formel

$$-X^1-(A^1)_m-\underset{\underset{O}{\|}}{C}-X^2-$$

stehen,

A für Sauerstoff oder für einen Rest $>N-R^8$ steht und

X für Sauerstoff oder Schwefel steht, wobei

$R^7$ für Wasserstoff, Methyl, Ethyl, n- oder i-Propyl, Allyl, n- oder i-Butenyl, Propargyl, n- oder

i-Butinyl, Cyanmethyl, Ethoxyethyl, Ethoxyethoxyethyl, Methoxycarbonylmethyl, Ethoxycarbonylmethyl, Propoxycarbonylmethyl, Methoxycarbonylethyl, Ethoxycarbonylethyl, Propoxycarbonylethyl, Methoxycarbonylpropyl, Ethoxycarbonylpropyl, Propoxycarbonylpropyl, für Pyridyl, Pyridylmethyl oder Pyridylethyl steht,

$R^8$ für Wasserstoff, Methyl, Ethyl, n- oder i-Propyl, Fluormethyl, Chlormethyl, Brommethyl, Difluormethyl oder Trifluormethyl steht,

$A^1$ für einen der Reste $-CH_2-$,

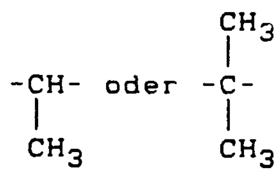

steht,

$X^1$ für Sauerstoff oder Schwefel steht,

$X^2$ für Sauerstoff, Schwefel oder $>N-R^7$ steht und

m für eine Zahl 0 oder 1 steht.

Im einzelnen seien außer den bei den Herstellungsbeispielen genannten Verbindungen die folgenden arylsubstituierten Alkylidenstickstoffheterocyclen der allgemeinen Formel (I) genannt:

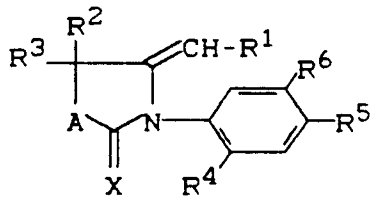

(I)

| $R^1$ | $R^2$ | $R^3$ | $R^4$ | $R^5$ | $R^6$ | A | X |
|---|---|---|---|---|---|---|---|
| H | H | H | H | Cl | $-O-CH_2CH=CH_2$ | O | O |
| H | H | H | F | Cl | $-OCH_2C≡CH$ | NH | O |
| H | H | H | F | Cl | $-SCH_2COOC_2H_5$ | $N-CH_3$ | O |
| $CH_3$ | H | H | F | Cl | $-OCH_2C≡CH$ | $N-CH_3$ | O |
| H | H | H | F | Br | $-SCH_2COOC_2H_5$ | O | O |
| H | H | H | F | Br | $-SCHCOOC_2H_5$<br>$CH_3$ | $N-CH_3$ | O |

| $R^1$ | $R^2$ | $R^3$ | $R^4$ | $R^5$ | $R^6$ | A | X |
|---|---|---|---|---|---|---|---|
| $CH_3$ | H | H | F | Br | $-SCH_2COOCH_3$ | NH | O |
| H | H | H | F | $CH_3$ | $-OCH_2C{\equiv}CH$ | O | O |
| H | H | H | F | $CH_3$ | $-SCH_2C{\equiv}CH$ | $N-CH_3$ | O |
| H | H | H | H | CN | $-OCH(CH_3)_2$ | O | S |
| $C_2H_5$ | H | H | F | CN | $-OCH_2CH=CH_2$ | NH | O |
| H | H | H | F | CN | $-SCH_2COOC_2H_5$ | $N-CH_3$ | O |
| H | H | H | F | Cl | $-OCH_2C{\equiv}CH$ | $N-CH_3$ | O |
| H | H | H | F | CN | $-SCHCOOC_2H_5$<br>\|<br>$CH_3$ | O | O |
| H | H | H | F | Cl | $-SCHCOOC_2H_5$<br>\|<br>$CH_3$ | O | O |
| H | H | $CH_3$ | H | Cl | $-OCH_2COOC_2H_5$ | O | O |
| H | H | $CH_3$ | F | Cl | $-OCH_2COOCH_3$ | O | S |
| H | H | $CH_3$ | H | Cl | $-OCHCOOC_2H_5$<br>\|<br>$CH_3$ | O | O |
| H | H | $CH_3$ | F | Cl | $-OCHCOOC_2H_5$<br>\|<br>$CH_3$ | O | O |
| H | H | $CH_3$ | H | Br | $-OCH_2COOC_3H_7$ | O | O |
| H | H | $CH_3$ | F | Br | $-OCH_2COOC_2H_5$ | O | S |
| H | H | $CH_3$ | F | Br | $-OCHCOOC_2H_5$<br>\|<br>$CH_3$ | O | O |

| $R^1$ | $R^2$ | $R^3$ | $R^4$ | $R^5$ | $R^6$ | A | X |
|---|---|---|---|---|---|---|---|
| H | H | 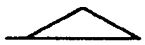 | F | Cl | $-OCH_2C{\equiv}CH$ | O | O |
| H | H | $CH_3$ | F | Cl | $-O(CH_2CH_2O)_2C_2H_5$ | O | O |
| H | H | $CH_3$ | F | Cl | $-OCHC{\equiv}CH$<br>$CH_3$ | O | O |
| $CH_3$ | H | $CH_3$ | F | Br | $-OCH_2C{\equiv}CH$ | O | O |
| H | H | $C_2H_5$ | F | Br | $-OCH_2CH{=}CH_2$ | O | O |
| H | H | $CH_3$ | H | CN | $-OCH_2COOC_2H_5$ | O | O |
| H | H | $n-C_3H_7$ | F | Br | $-OCH_2CN$ | O | O |
| H | H | $CH_3$ | F | $CH_3$ | $-OCHC{\equiv}CN$<br>$CH_3$ | O | O |
| $C_6H_5$ | H | $CH_3$ | F | $CH_3$ | $-OCH(CH_3)_2$ | O | O |
| H | H | $CH_3$ | F | CN | $-OCH(CH_3)_2$ | O | O |
| H | H | $CH_3$ | F | CN | $-OCH_2C{\equiv}CH$ | O | O |
| H | H | $CH_3$ | F | CN | $-OCH_2C{\equiv}CH$ | O | S |
| H | H | $CH_3$ | F | CN | $-OCH_2COOC_2H_5$ | O | O |
| $C_2H_5$ | H | $CH_3$ | F | CN | $-OCH_3$ | O | O |
| $CH_3$ | H | $CH_3$ | F | Cl | 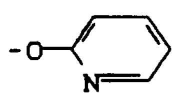 | O | O |
| H | H | $CH_3$ | H | Cl | $-O-CH_2COOC_2H_5$ | NH | O |
| $CH_3$ | H | $CH_3$ | H | Cl | $-O-CH_2COOC_2H_5$ | $N-CH_3$ | O |
| Cl | H | $CH_3$ | F | Cl | $-O-CH_2COOCH_3$ | $N-CH_3$ | S |

| $R^1$ | $R^2$ | $R^3$ | $R^4$ | $R^5$ | $R^6$ | A | X |
|---|---|---|---|---|---|---|---|
| H | H | $CH_3$ | H | Cl | $-O-CHCOOC_2H_5$<br>$C_2H_5$ | NH | O |
| H | H | $CH_3$ | F | Cl | $-O-CHCOOC_2H_5$<br>$CH_3$ | NH | O |
| H | H | $CH_3$ | F | Cl | $-O-CH(CH_3)COOC_2H_5$ | $N-CH_3$ | O |
| H | H | $CH_3$ | H | Br | $-O-CH_2COOC_3H_7$ | $N-CH_3$ | O |
| H | H | $CH_3$ | F | Br | $-OCHCOOC_2H_5$<br>$CH_3$ | $N-C_2H_5$ | O |
| H | H | $CH_3$ | F | Br | $-OCH(CH_3)COOCH_3$ | $N-CH_3$ | O |
| H | H | $CH_3$ | F | Cl | $-OCH_2C{\equiv}CH$ | NH | O |
| H | H | $CH_3$ | F | Cl | $-OCH_2C{\equiv}CH$ | $N-CH_3$ | O |
| H | H | $CH_3$ | F | Cl | $-OCHC{\equiv}CH$<br>$CH_3$ | NH | O |
| $n-C_3H_7$ | H | $CH_3$ | F | Br | $-OCH_2C{\equiv}CH$ | $N-CH_3$ | O |
| $n-C_3H_7$ | H | $CH_3$ | F | Br | $-OCH_2C{\equiv}CH$ | NH | O |
| $CH_3$ | H | $CH_3$ | F | Br | $-OCH_2C{\equiv}CH$ | $N-CH_3$ | O |
| H | H | $C_2H_5$ | F | Br | $-OCH_2CH=CH_2$ | $N-C_2H_5$ | O |
| H | H | $CH_3$ | H | CN | $-OCH_2COOC_2H_5$ | $N-CH_3$ | O |
| H | H | $n-C_3H_7$ | F | Br | $-OCH_2CN$ | NH | O |
| H | H | $CH_3$ | F | $CH_3$ | $-OCHC{\equiv}CH$<br>$CH_3$ | $N-C_2H_5$ | O |
| H | H | $CH_3$ | F | $CH_3$ | $-OCH_2C{\equiv}CH$ | $N-CH_3$ | S |

| $R^1$ | $R^2$ | $R^3$ | $R^4$ | $R^5$ | $R^6$ | A | X |
|---|---|---|---|---|---|---|---|
| $C_2H_5$ | H | $CH_3$ | F | $CH_3$ | $-OCH(CH_3)_2$ | $N-C_2H_5$ | O |
| H | H | $CH_3$ | F | CN | $-OCH(CH_3)_2$ | $N-CH_3$ | O |
| H | H | $CH_3$ | F | CN | $-OCH_2C\equiv CH$ | NH | O |
| H | H | $CH_3$ | F | CN | $-OCH_2CH=CH_2$ | $N-CH_3$ | O |
| H | H | $CH_3$ | F | CN | $-OCH_2COOC_2H_5$ | NH | O |
| H | H | $CH_3$ | F | CN | $-OCHCOOC_2H_5$<br>$CH_3$ | $N-CH_3$ | O |
| $C_2H_5$ | H | $CH_3$ | F | CN | $-OCH_3$ | $N-C_3H_7$ | O |
| $CH_3$ | H | $CH_3$ | F | CN | 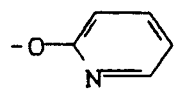 | $N-CH_3$ | O |
| H | H | $CH_3$ | H | Cl | $-SCH_2COOC_2H_5$ | O | O |
| H | H | $CH_3$ | H | Cl | $-SCHCOOC_2H_5$<br>$CH_3$ | O | O |
| H | H | $CH_3$ | F | Cl | $-SCHCOOC_2H_5$<br>$CH_3$ | O | O |
| H | H | $CH_3$ | H | Br | $-SCH_2COOC_3H_7$ | O | O |
| H | H | $CH_3$ | F | Br | $-SCHCOOC_2H_5$<br>$CH_3$ | O | O |
| H | H | $CH_3$ | F | Cl | $-SCH_2C\equiv CH$ | O | O |
| H | H | $CH_3$ | F | Cl | $-SCH_2C\equiv CH$ | O | S |
| H | H | $CH_3$ | F | Cl | $-SCHC\equiv CH$<br>$CH_3$ | O | O |

| $R^1$ | $R^2$ | $R^3$ | $R^4$ | $R^5$ | $R^6$ | A | X |
|---|---|---|---|---|---|---|---|
| $CH_3$ | H | $CH_3$ | F | Br | $-SCH_2C\equiv CH$ | O | O |
| H | H | $C_2H_5$ | F | Br | $-SCH_2CH=CH_2$ | O | O |
| H | H | $CH_3$ | H | CN | $-SCH_2COOC_2H_5$ | O | O |
| H | H | $n-C_3H_7$ | F | Br | $-SCH_2CN$ | O | O |
| H | H | $CH_3$ | F | $CH_3$ | $-SCHC\equiv CH$<br>\|<br>$CH_3$ | O | O |
| $C_6H_5$ | H | $CH_3$ | F | $CH_3$ | $-SCH(CH_3)_2$ | O | O |
| $CH_3$ | H | $CH_3$ | F | $CH_3$ | $-SCH_2CN$ | O | S |
| H | H | $CH_3$ | F | CN | $-SCH(CH_3)_2$ | O | O |
| H | H | $CH_3$ | F | CN | $-SCH_2C\equiv CH$ | O | O |
| H | H | $CH_3$ | F | CN | $-SCH_2COOC_2H_5$ | O | O |
| $C_2H_5$ | H | $CH_3$ | F | CN | $-SCH_3$ | O | O |
| H | H | $CH_3$ | H | Cl | $-SCH_2COOC_2H_5$ | NH | O |
| H | H | $CH_3$ | H | Cl | $-SCH_2COOC_2H_5$ | $N-CH_3$ | S |
| H | H | $CH_3$ | F | Cl | $-SCH_2COOC_2H_5$ | $N-CH_3$ | O |
| H | H | $CH_3$ | H | Cl | $-SCHCOOC_2H_5$<br>\|<br>$CH_3$ | NH | O |
| H | H | $CH_3$ | F | Cl | $-SCHCOOC_2H_5$<br>\|<br>$CH_3$ | NH | O |
| H | H | $CH_3$ | F | Cl | $-SCH(CH_3)COOC_2H_5$ | $N-CH_3$ | O |
| H | H | $CH_3$ | H | Br | $-SCH_2COOC_3H_7$ | NH | O |
| H | H | $CH_3$ | F | Br | $-SCHCOOC_2H_5$<br>\|<br>$CH_3$ | NH | O |

| $R^1$ | $R^2$ | $R^3$ | $R^4$ | $R^5$ | $R^6$ | A | X |
|---|---|---|---|---|---|---|---|
| H | H | $CH_3$ | F | Br | $-SCHCOOC_2H_5$<br>\|<br>$CH_3$ | $N-CH_3$ | O |
| H | H | $CH_3$ | F | Br | $-SCH(CH_3)COOC_2H_5$ | $N-CH_3$ | O |
| H | H | $CH_3$ | F | Cl | $-SCH_2C{\equiv}CH$ | NH | O |
| H | H | $CH_3$ | F | Cl | $-SCH_2C{\equiv}CH$ | $N-C_2H_5$ | O |
| H | H | $CH_3$ | F | Cl | $-SCHC{\equiv}CH$<br>\|<br>$CH_3$ | NH | O |
| H | H | $CH_3$ | F | Cl | $-SCH(CH_3)C{\equiv}CH$ | $N-CH_3$ | O |
| $CH_3$ | H | $CH_3$ | F | Br | $-SCH_2C{\equiv}CH$ | NH | O |
| $CH_3$ | H | $CH_3$ | F | Br | $-SCH_2C{\equiv}CH$ | $N-CH_3$ | O |
| H | H | $C_2H_5$ | F | Br | $-SCH_2CH=CH_2$ | $N-C_2H_5$ | O |
| H | H | $CH_3$ | H | CN | $-SCH_2COOC_2H_5$ | NH | O |
| H | H | $CH_3$ | F | CN | $-SCHCOOC_2H_5$<br>\|<br>$CH_3$ | $N-CH_3$ | O |
| H | H | $n-C_3H_7$ | F | Br | $-SCH_2CN$ | $N-CHF_2$ | O |
| H | H | $CH_3$ | F | $CH_3$ | $-SCHC{\equiv}CH$<br>\|<br>$CH_3$ | $N-CH_3$ | O |
| H | H | $CH_3$ | F | $CH_3$ | $-SCH_2CH=CH_2$ | $N-CH_3$ | S |
| $C_6H_5$ | H | $CH_3$ | F | $CH_3$ | $-SCH(CH_3)_2$ | $N-C_3H_7$ | O |
| H | H | $CH_3$ | F | CN | $-SCH(CH_3)_2$ | NH | O |
| H | H | $CH_3$ | F | CN | $-SCH_2C{\equiv}CH$ | $N-CH_3$ | O |
| H | H | $CH_3$ | F | CN | $-SCH_2COOC_2H_5$ | NH | S |

| $R^1$ | $R^2$ | $R^3$ | $R^4$ | $R^5$ | $R^6$ | A | X |
|---|---|---|---|---|---|---|---|
| H | H | $CH_3$ | F | CN | $-SCH_2COOC_2H_5$ | $N-CH_3$ | O |
| $C_2H_5$ | H | $CH_3$ | F | CN | $-SCH_3$ | $N-C_2H_5$ | O |
| H | H | H | H | $-O-CH_2-C-N-$<br>‖ \|<br>O $CH_3$ | | O | O |
| H | H | H | F | $-O-CH_2-C-N-$<br>‖ \|<br>O $CH_2-C{\equiv}CH$ | | O | O |
| H | H | H | F | $-O-CH_2-C-N-$<br>‖ \|<br>O $CH_2-C{\equiv}CH$ | | $N-CH_3$ | O |
| H | H | H | F | $-S-CH_2-C-N-$<br>‖ \|<br>O $CH_2-CH=CH_2$ | | O | O |
| $CH_3$ | H | H | F | $-S-C-N-$<br>‖ \|<br>O $CH_2-C{\equiv}CH$ | | $N-CH_3$ | O |
| H | H | H | F | $-S-C-N-$<br>‖ \|<br>O $CH_2-C{\equiv}CH$ | | NH | O |
| H | H | H | F | $-S-C-N-$<br>‖ \|<br>O $CH_2-C{\equiv}CH$ | | $N-CH_3$ | O |
| H | H | H | F | $-O-CH(CH_3)-C-N-$<br>‖ \|<br>O $CH_2CN$ | | O | O |
| Cl | H | H | F | $-O-CH_2-C-N-$<br>‖ \|<br>O $CH_2-C{\equiv}CH$ | | O | O |
| H | H | H | F | $-S-C-N-$<br>‖ \|<br>O $CH_2-C{\equiv}CH$ | | O | O |

| $R^1$ | $R^2$ | $R^3$ | $R^4$ | $R^5$ | $R^6$ | A | X |
|---|---|---|---|---|---|---|---|
| H | H | H | F | $-O-CH_2-C-N-$<br>‖ \|<br>$O\ CH_2-C\equiv CH$ | | $N-CH_3$ | O |
| $CH_3$ | H | H | F | $-S-C-N-$<br>‖ \|<br>$O\ CH_2-CH=CH_2$ | | O | O |
| H | H | $CH_3$ | H | $-O-CH_2-C-N-$<br>‖ \|<br>$O\ CH_2-C-N-(CH_3)_2$<br>‖<br>O | | O | O |
| H | H | $n-C_3H_7$ | F | $-O-CH(CH_3)-C-N-$<br>‖ \|<br>$O\ CH_2CN$ | | O | O |
| H | H | $CH_3$ | F | $-O-C-N-$<br>‖ \|<br>$O\ CH_3$ | | $N-CH_3$ | O |
| H | H | $CH_3$ | F | $-S-C-N-$<br>‖ \|<br>$O\ CH_2C\equiv CH$ | | $N-CH_3$ | O |
| $C_6H_5$ | H | $CH_3$ | F | $-O-CH_2-C-N-$<br>‖ \|<br>$O\ CH_2-COOC_2H_5$ | | O | O |
| H | H | $C_2H_5$ | F | $-O-CH(CH_3)-C-N-$<br>‖ \|<br>$O\ CH_2-C\equiv CH$ | | O | S |
| H | H | $CH_3$ | F | $-O-C(CH_3)_2-C-N-$<br>‖ \|<br>$O\ C_2H_5$ | | O | O |
| $CH_3$ | H | $CH_3$ | F | $-O-CH_2-C-N-$<br>‖ \|<br>$O\ CH_2-COOCH_3$ | | O | O |

| $R^1$ | $R^2$ | $R^3$ | $R^4$ | $R^5$ | $R^6$ | A | X |
|---|---|---|---|---|---|---|---|
| $C_2H_5$ | H | $CH_3$ | F | $-S-CH_2-C-N-$<br>‖ \|<br>O $CH_2CN$ | | O | O |
| H | H | $CH_3$ | F | 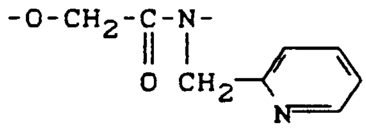 | | O | O |
| H | H | $CH_3$ | H | $-O-CH_2-C-N-$<br>‖ \|<br>O $CH_2-CH=CH_2$ | | O | O |
| H | H | $CH_3$ | F | $-O-C-N-$<br>‖ \|<br>O $CH_2-C\equiv CH$ | | NH | O |
| H | H | $CH_3$ | F | $-O-CH_2-C-N-$<br>‖ \|<br>O $CH_2C\equiv CH$ | | $N-CH_3$ | O |
| H | H | $CH_3$ | F | $-S-C-N-$<br>‖ \|<br>O $CH_2-C\equiv CH$ | | O | O |
| H | H | $CH_3$ | F | $-S-C-N-$<br>‖ \|<br>O $CH_2-C\equiv CH$ | | NH | O |
| $CH_3$ | H | $CH_3$ | F | $-O-CH(CH_3)-C-N-$<br>‖ \|<br>O $CH_2-COOC_2H_5$ | | O | O |
| H | H | $CH_3$ | F | $-O-CH(CH_3)-C-N-$<br>‖ \|<br>O $CH_2-C\equiv CH$ | | O | O |
| H | H | $CH_3$ | F | $-O-CH(CH_3)-C-N-$<br>‖ \|<br>O $CH_2-C\equiv CH$ | | NH | O |
| H | H | $CH_3$ | F | $-O-CH_2-C-N-$<br>‖ \|<br>O $CH_2-CH=CH_2$ | | $N-CH_3$ | O |

| $R^1$ | $R^2$ | $R^3$ | $R^4$ | $R^5$ | $R^6$ | A | X |
|---|---|---|---|---|---|---|---|
| H | H | $CH_3$ | F | 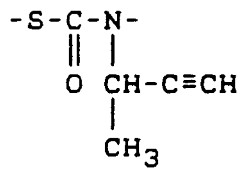 | | O | O |
| Cl | H | $C_2H_5$ | F | 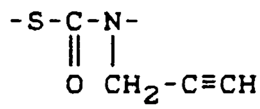 | | $N-CH_3$ | O |
| $CH_3$ | H | $CH_3$ | F | 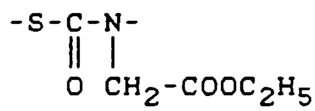 | | O | O |
| H | $CH_3$ | $CH_3$ | F | 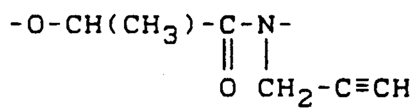 | | O | O |
| H | $-(CH_2)_2-$ | | F | 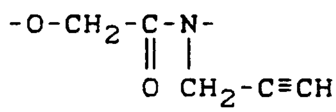 | | O | O |
| H | $CH_3$ | $CH_3$ | H | 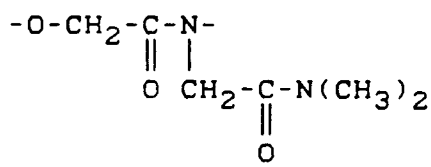 | | O | S |
| H | $-(CH_2)_2-$ | | F | 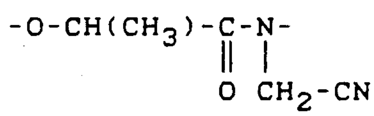 | | O | O |
| H | $CH_3$ | $CH_3$ | F | 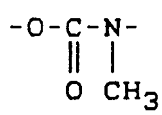 | | $N-CH_3$ | O |
| H | H | $CH_3$ | F | 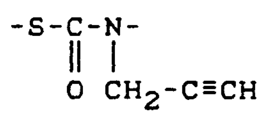 | | $N-CH_3$ | O |
| $C_6H_5$ | $CH_3$ | $CH_3$ | F | 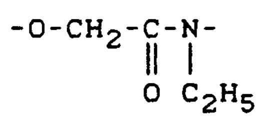 | | O | O |

| $R^1$ | $R^2$ | $R^3$ | $R^4$ | $R^5$ | $R^6$ | A | X |
|---|---|---|---|---|---|---|---|
| H | $-(CH_2)_2-$ | | F | | 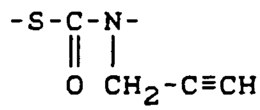 | O | O |
| H | $-(CH_2)_3-$ | | F | | 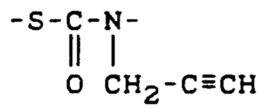 | $N-CH_3$ | O |
| H | $CH_3$ | $CH_3$ | F | | 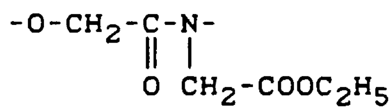 | NH | O |
| $CH_3$ | $-(CH_2)_2-$ | | F | | 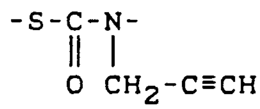 | O | O |
| H | $CH_3$ | $C_2H_5$ | F | | 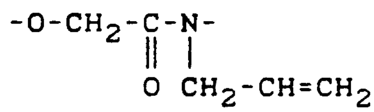 | $N-CH_3$ | O |
| $C_2H_5$ | $CH_3$ | $CH_3$ | F | | 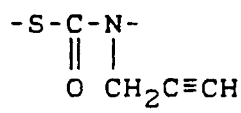 | O | O |
| $CH_3$ | $-(CH_2)_4-$ | | F | | 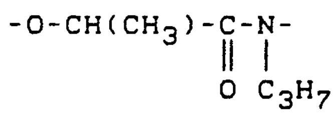 | O | S |
| H | $CH_3$ | $CH_3$ | H | Cl | $-OCH_2COOC_2H_5$ | O | O |
| $CH_3$ | $CH_3$ | $CH_3$ | F | Cl | $-OCH(CH_3)COOCH_3$ | O | O |
| Cl | $CH_3$ | $CH_3$ | F | Cl | $-OCH_2COOC_2H_5$ | O | O |
| H | $CH_3$ | $CH_3$ | H | Cl | 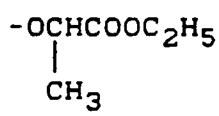 | NH | S |
| H | $CH_3$ | $CH_3$ | F | Cl | $-OCH_2COOC_3H_7$ | $N-CH_3$ | O |
| H | $CH_3$ | $CH_3$ | F | Cl | 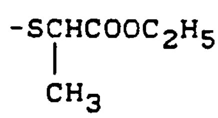 | $N-CH_3$ | O |

| $R^1$ | $R^2$ | $R^3$ | $R^4$ | $R^5$ | $R^6$ | A | X |
|---|---|---|---|---|---|---|---|
| H | $-(CH_2)_2-$ | | F | Cl | $-SCH_2COOC_2H_5$ | O | O |
| H | $CH_3$ | $CH_3$ | H | Br | $-SCH_2COOC_3H_7$ | O | O |
| H | $CH_3$ | $n-C_3H_7$ | F | Br | $-SCH_2COOCH_3$ | $N-CH_3$ | S |
| H | $-(CH_2)_2-$ | | F | Br | $-SCH_2COOC_2H_5$ | $N-CH_3$ | O |
| H | $CH_3$ | $CH_3$ | F | Br | $-SCHCOOC_2H_5$<br>$\vert$<br>$CH_3$ | $N-CH_3$ | O |
| H | $CH_3$ | $CH_3$ | F | Cl | $-OCH_2C{\equiv}CH$ | O | O |
| H | $CH_3$ | $C_2H_5$ | F | Br | $-SCH_2C{\equiv}CH$ | O | S |
| H | $CH_3$ | $CH_3$ | F | Cl | $-SCHC{\equiv}CH$<br>$\vert$<br>$CH_3$ | O | O |
| H | $-(CH_2)_2-$ | | F | Cl | $-OCH_2C{\equiv}CH$ | O | O |
| $CH_3$ | $CH_3$ | $CH_3$ | F | Br | $-SCH_2C{\equiv}CH$ | O | O |
| $CH_3$ | $CH_3$ | $CH_3$ | F | Br | $-SCH_2C{\equiv}CH$ | $N-CH_3$ | O |
| H | $CH_3$ | $C_2H_5$ | F | Br | $-SCH_2CH{=}CH_2$ | O | O |
| H | $CH_3$ | $CH_3$ | F | CN | $-OCH_2CH{=}CH_2$ | $N-CH_3$ | O |
| H | $CH_3$ | $CH_3$ | H | CN | $-SCH_2COOC_2H_5$ | O | O |
| H | $CH_3$ | $n-C_3H_7$ | F | Br | $-SCH_2CN$ | O | O |
| H | $CH_3$ | $CH_3$ | F | $CH_3$ | $-OCH_2COOC_2H_5$ | O | O |
| H | $CH_3$ | $CH_3$ | F | $CH_3$ | $-SCHC{\equiv}CH$<br>$\vert$<br>$CH_3$ | O | O |
| Cl | $CH_3$ | $CH_3$ | F | $CH_3$ | $-OCH(CH_3)_2$ | $N-CH_3$ | O |

| $R^1$ | $R^2$ | $R^3$ | $R^4$ | $R^5$ | $R^6$ | A | X |
|---|---|---|---|---|---|---|---|
| $C_6H_5$ | $CH_3$ | $CH_3$ | F | $CH_3$ | $-SCH(CH_3)_2$ | O | O |
| H | $-(CH_2)_2-$ | | F | $CH_3$ | $-OCH_2C{\equiv}CH$ | O | O |
| H | $CH_3$ | $CH_3$ | F | CN | $-OCH(CH_3)_2$ | O | O |
| H | $CH_3$ | $CH_3$ | H | CN | $-SCH(CH_3)_2$ | $N-CH_3$ | O |
| H | $CH_3$ | $CH_3$ | F | CN | $-SCH_2C{\equiv}CH$ | O | O |
| H | $-(CH_2)_2-$ | | F | CN | $-OCH_2CH{=}CH_2$ | O | O |
| H | $CH_3$ | $CH_3$ | F | CN | $-SCH_2COOC_2H_5$ | O | O |
| H | $-(CH_2)_3-$ | | F | Cl | $-OCH_2C{\equiv}CH$ | $N-CH_3$ | O |
| $C_2H_5$ | $CH_3$ | $CH_3$ | F | CN | $-SCH_3$ | O | O |
| H | $CH_3$ | $CH_3$ | F | Cl | 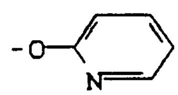 | O | O |

Verwendet man beispielsweise 2-Fluor-4-chlor-5-propargyloxyphenylisocyanat und Propargylalkohol als Ausgangsstoffe, so läßt sich der Reaktionsablauf des erfindungsgemäßen Verfahrens (a) durch das folgende Formelschema darstellen:

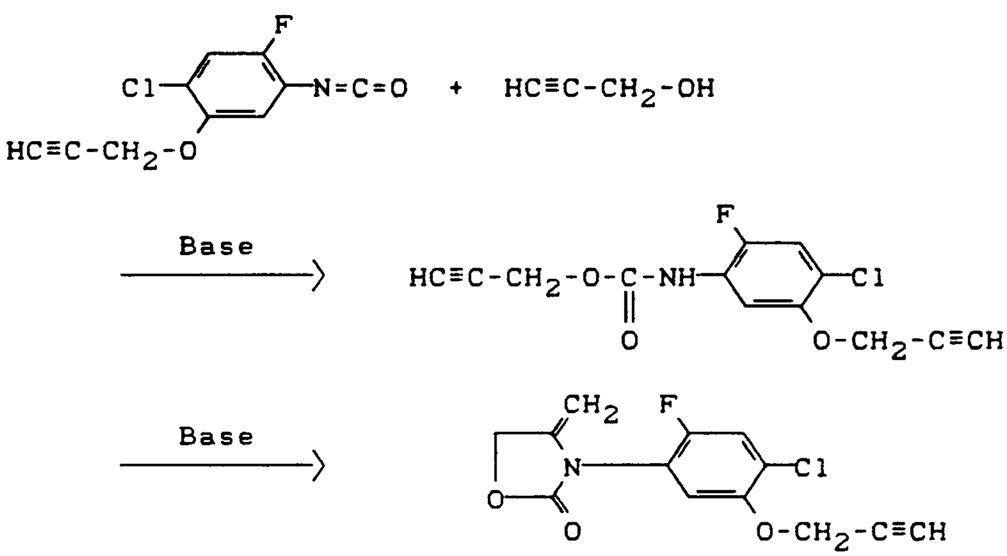


Verwendet man beispielsweise 4-Chlor-2-fluor-5-isopropoxyanilin und 3-Methyl-3-butinol sowie Kohlendioxid als Ausgangsstoffe, so läßt sich der Reaktionsablauf des erfindungsgemäßen Verfahrens (b) durch das folgende Formelschema darstellen:

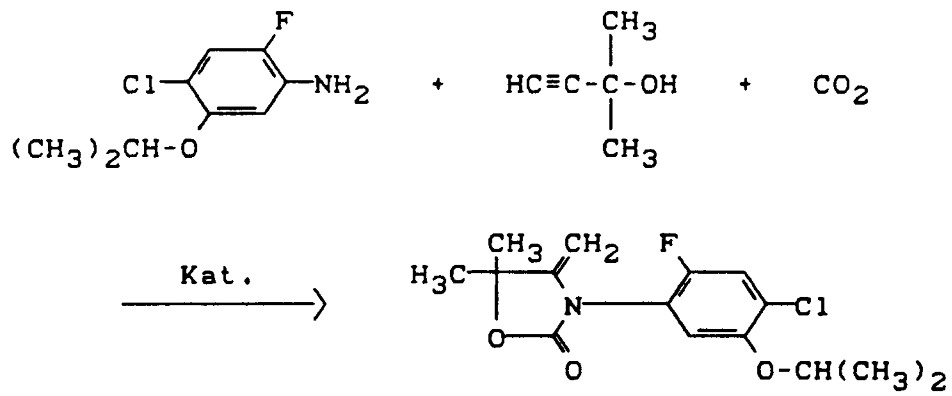


Verwendet man beispielsweise 3-(4-Cyano-2-fluor-5-allyloxyphenyl)-5-methyl-4-methylidenimidazolidin-2-on und Ethyliodid als Ausgangsstoffe, so läßt sich der Reaktionsablauf des erfindungsgemäßen Verfahrens (c) durch das folgende Formelschema darstellen:

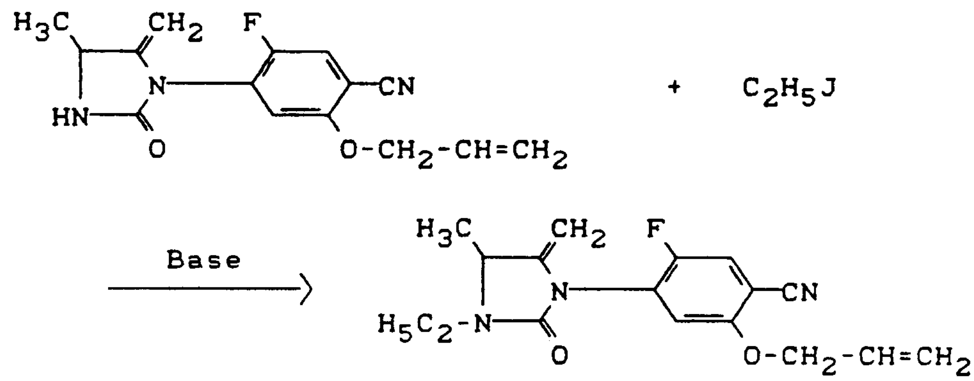


Die zur Durchführung des erfindungsgemäßen Verfahrens (a) als Ausgangsstoffe benötigten Alkinylverbindungen sind durch die Formel (II) allgemein definiert. In dieser Formel (II) stehen $R^1$, $R^2$, $R^3$ und A vorzugsweise für diejenigen Reste, die bereits im Zusammenhang mit der Beschreibung der erfindungsgemäßen Stoffe der Formel (I) als bevorzugt für diese Substituenten genannt wurden.

Die Alkinylverbindungen der Formel (II) sind allgemein bekannte Verbindungen der organischen Chemie oder erhältlich in Analogie zu allgemein bekannten Verfahren (vergleiche z. B. Houben Weyl, Methoden der organischen Chemie, Band 6/1 a/1; Journal of Amer. Chem. Soc. 75, 1653 [1953]).

Die zur Durchführung des erfindungsgemäßen Verfahrens (a) weiterhin als Ausgangsstoffe benötigten Iso(thio)cyanate sind durch die Formel (III) allgemein definiert. In dieser Formel (III) stehen $R^4$, $R^5$, $R^6$ und X vorzugsweise für diejenigen Reste, die bereits im Zusammenhang mit der Beschreibung der erfindungsgemäßen Stoffe der Formel (I) als bevorzugt für diese Substituenten genannt wurden.

Die Iso(thio)cyanate der Formel (III) sind bekannt oder erhältlich in Analogie zu bekannten Verfahren (vergleiche z. B. EP 263 299; EP 255 601; EP 230 874; EP 304 920; EP 312 064).

Die bei der Durchführung des erfindungsgemäßen Verfahrens (a) als Zwischenprodukt auftretenden Alkinylcarbamate (oder -harnstoffe) sind durch die Formel (IV) allgemein definiert. In dieser Formel (IV) stehen $R^1$, $R^2$, $R^3$, $R^4$, $R^5$, $R^6$, A und X vorzugsweise für diejenigen Reste, die bereits im Zusammenhang mit der Beschreibung der erfindungsgemäßen Stoffe der Formel (I) als bevorzugt für diese Substituenten genannt wurden.

Die Alkinylcarbamate und -harnstoffe der Formel (IV) sind noch nicht bekannt und ebenfalls Gegenstand der Erfindung.

Die zur Durchführung des erfindungsgemäßen Verfahrens (b) als Ausgangsstoffe benötigten Alkinylverbindungen sind durch die Formel (IIa) allgemein definiert. In dieser Formel (IIa) stehen $R^1$, $R^2$ und $R^3$ vorzugsweise für diejenigen Reste, die bereits im Zusammenhang mit der Beschreibung der erfindungsgemäßen Stoffe der Formel (I) als bevorzugt für diese Substituenten genannt wurden.

Die Alkinylverbindungen der Formel (IIa) sind allgemein bekannte Verbindungen der organischen Chemie.

Die zur Durchführung des erfindungsgemäßen Verfahrens (b) weiterhin als Ausgangsstoffe benötigten Anilinderivate sind durch die Formel (V) allgemein definiert. In dieser Formel (V) stehen $R^4$, $R^5$ und $R^6$ vorzugsweise für diejenigen Reste, die bereits im Zusammenhang mit der Beschreibung der erfindungsgemäßen Stoffe der Formel (I) als bevorzugt für diese Substituenten genannt wurden.

Die Anilinderivate der Formel (V) sind bekannt oder erhältlich in Analogie zu bekannten Verfahren (vergleiche z. B. EP 237 899; EP 170 191; EP 218 972; EP 126 419; EP 142 648).

Die zur Durchführung des erfindungsgemäßen Verfahrens (c) als Ausgangsstoffe benötigten arylsubstituierten Alkylidenstickstoffheterocyclen sind durch die Formel (Ia) allgemein definiert` In dieser Formel (Ia) stehen $R^1$, $R^2$, $R^3$, $R^4$, $R^5$, $R^6$ und X vorzugsweise für diejenigen Reste, die bereits im Zusammenhang mit der Beschreibung der erfindungsgemäßen Stoffe der Formel (I) als bevorzugt für diese Substituenten genannt wurden.

Die arylsubstituierten Alkylidenstickstoffheterocyclen der Formel (Ia) sind erfindungsgemäße Verbindungen und erhältlich mit Hilfe des erfindungsgemäßen Verfahrens (a).

Die zur Durchführung des erfindungsgemäßen Verfahrens (c) weiterhin als Ausgangsstoffe benötigten Alkylierungsmittel sind durch die Formel (VI) allgemein definiert. In dieser Formel (VI) steht $R^{8-1}$ vorzugsweise für diejenigen Reste, die bereits im Zusammenhang mit der Beschreibung der erfindungsgemäßen Stoffe der Formel (I) als bevorzugt für den Substituenten $R^8$ genannt wurden, mit Ausnahme des Wasserstoffrestes.

E steht für einen bei Alkylierungsmitteln üblichen Abgangsrest, vorzugsweise für Halogen, insbesondere für Chlor, Brom oder Iod, oder für jeweils gegebenenfalls substituiertes Alkylsulfonyloxy mit vorzugsweise 1 bis 4 Kohlenstoffatomen, Alkoxysulfonyloxy mit vorzugsweise 1 bis 4 Kohlenstoffatomen oder Arylsulfonyloxy mit vorzugsweise 6 bis 10 Kohlenstoffatomen, wie insbesondere Methansulfonyloxy, Trifluormethansulfonyloxy, Methoxysulfonyloxy, Ethoxysulfonyloxy oder p-Toluolsulfonyloxy.

Die Alkylierungsmittel der Formel (VI) sind allgemein bekannte Verbindungen der organischen Chemie.

Als Verdünnungsmittel zur Durchführung des erfindungsgemäßen Verfahrens (a) kommen inerte organische Lösungsmittel infrage. Hierzu gehören insbesondere aliphatische, alicyclische oder aromatische, gegebenenfalls halogenierte Kohlenwasserstoffe wie beispielsweise Benzin, Benzol, Toluol, Xylol, Chlorbenzol, Petrolether, Hexan, Cyclohexan, Dichlormethan, Chloroform, Tetrachlorkohlenstoff, Ether, wie Diethylether, Dioxan, Tetrahydrofuran oder Ethylenglykoldimethyl- oder -diethylether, Ketone, wie Aceton oder Butanon, Nitrile, wie Acetonitril oder Propionitril, Amide, wie Dimethylformamid, Dimethylacetamid, N-Methylformanilid, N-Methylpyrrolidon oder Hexamethylphosphorsäuretriamid, Ester, wie Essigsäureethylester oder Sulfoxide, wie Dimethylsulfoxid.

Das erfindungsgemäße Verfahren (a) wird vorzugsweise in Gegenwart eines geeigneten Reaktionshilfsmittels durchgeführt. Als solche kommen alle üblicherweise verwendbaren anorganischen und organischen Basen infrage. Vorzugsweise verwendet man Alkalimetallhydride, -hydroxide, -amide, -alkoholate, -carbonate oder -hydrogencarbonate, wie beispielsweise Natriumhydrid, Natriumamid, Natriumhydroxid, Natriummethylat, Natriumethylat, Kalium-t-butylat, Natriumcarbonat oder Natriumhydrogencarbonat oder auch tertiäre Amine, wie beispielsweise Triethylamin, N,N-Dimethylanilin, Pyridin, N,N-Dimethylaminopyridin, Diazabicyclooctan (DABCO), Diazabicyclononen (DBN) oder Diazabicycloundecen (DBU).

Die Reaktionstemperaturen können bei der Durchführung des erfindungsgemäßen Verfahrens (a) in einem größeren Bereich variiert werden, Im allgemeinen arbeitet man bei Temperaturen zwischen 0 °C und 150 °C, vorzugsweise bei Temperaturen zwischen 20 °C und 120 °C.

Zur Durchführung des erfindungsgemäßen Verfahrens (a) setzt man pro Mol an Alkinylverbindung der Formel (II) im allgemeinen 0.5 bis 5.0 Mol, vorzugsweise 0.8 bis 1.5 Mol an Iso(thio)cyanat der Formel (III) und gegebenenfalls 1.0 bis 10.0 Mol, vorzugsweise 1.0 bis 5.0 Mol an Reaktionshilfsmittel ein.

Die Reaktionsdurchführung, Aufarbeitung und Isolierung der Reaktionsprodukte erfolgt nach allgemein üblichen Methoden.

Dabei ist es möglich, das erfindungsgemäße Verfahren (a) in zwei separaten Reaktionsstufen durchzuführen, die intermediär auftretenden Zwischenprodukte der Formel (IV) zu isolieren und in einer folgenden, getrennten Reaktion mit Basen zu cyclisieren. Ebenso ist es jedoch auch möglich, die erforderliche Menge an als Reaktionshilfsmittel verwendeter Base gleich zu Anfang zuzusetzen, die Reaktionszeit entsprechend zu verlängern und beide Umsetzungen in einem Schritt in einer sogenannten Eintopfreaktion durchzuführen (vergleiche auch die Herstellungsbeispiele).

Als Verdünnungsmittel zur Durchführung des erfindungsgemäßen Verfahrens (b) kommen inerte organische Lösungsmittel infrage. Hierzu gehören insbesondere aliphatische, alicyclische oder aromatische, gegebenenfalls halogenierte Kohlenwasserstoffe wie beispielsweise Benzin, Benzol, Toluol, Xylol, Chlorbenzol, Petrolether, Hexan, Cyclohexan, Dichlormethan, Chloroform, Tetrachlorkohlenstoff, Ether, wie Diethylether, Dioxan, Tetrahydrofuran oder Ethylenglykoldimethyl- oder -diethylether, Ketone, wie Aceton oder Butanon, Nitrile, wie Acetonitril oder Propionitril, Amide, wie Dimethylformamid, Dimethylacetamid, N-Methylformanilid, N-Methylpyrrolidon oder Hexamethylphosphorsäuretriamid, Ester, wie Essigsäureethylester oder Sulfoxide, wie Dimethylsulfoxid.

Es ist jedoch auch möglich, das erfindungsgemäße Verfahren (b) ohne Zusatz eines Verdünnungsmittels direkt in Substanz durchzuführen.

Das erfindungsgemäße Verfahren (b) wird vorzugsweise in Gegenwart eines geeigneten Reaktionshilfsmittels durchgeführt. Als solche verwendet man insbesondere Kupfersalze, wie beispielsweise Kupfer-(I)-chlorid, Kupfer-(II)-formiat, Kupfer-(II)-acetat, Kupfer-(II)-sulfat oder andere Kupfersalze von organischen oder anorganischen Säuren oder auch mit Kupferionen beladene Ionenaustauscher.

Die Reaktionstemperaturen können bei der Durchführung des erfindungsgemäßen Verfahrens (b) in einem größeren Bereich variiert werden. Im allgemeinen arbeitet man bei Temperaturen zwischen 20 ° C und 180 ° C, vorzugsweise bei Temperaturen zwischen 50 ° C und 150 ° C.

Das erfindungsgemäße Verfahren (b) wird vorzugsweise unter Druck durchgeführt. Im allgemeinen arbeitet man in Druckbereichen zwischen 1 und 80 bar, vorzugsweise in Druckbereichen zwischen 20 und 50 bar.

Zur Durchführung des erfindungsgemäßen Verfahrens (b) setzt man pro Mol an Alkinylverbindung der Formel (IIa) im allgemeinen 0.5 bis 5.0 Mol, vorzugsweise 1.0 bis 3.0 Mol an Anilinderivat der Formel (V) und 1.0 bis 10.0 Mol, vorzugsweise 1.0 bis 5.0 Mol an gasförmigem Kohlendioxid, sowie 0.005 bis 1.0 Mol, vorzugsweise 0.01 bis 0.05 Mol an Kupferkatalysator ein.

Die Reaktionsdurchführung, Aufarbeitung und Isolierung der Reaktionsprodukte erfolgt nach bekannten Methoden (vergleiche z. B. DE 11 51 507).

Als Verdünnungsmittel zur Durchführung des erfindungsgemäßen Verfahrens (c) kommen inerte organische Lösungsmittel infrage. Hierzu gehören insbesondere aliphatische, alicyclische oder aromatische, gegebenenfalls halogenierte Kohlenwasserstoffe wie beispielsweise Benzin, Benzol, Toluol, Xylol, Pentan, Hexan, Heptan, Cyclohexan, Petrolether, Ligroin, Methylenchlorid, Chloroform, Tetrachlorkohlenstoff, Chlorbenzol oder Dichlorbenzol, Ether, wie Diethylether, Diisopropylether, Dioxan, Tetrahydrofuran oder Ethylenglykoldiethylether oder -dimethylether, Ketone, wie Aceton, Butanon, Methylisopropylketon oder Methylisobutylketon, Ester, wie Essigsäureethylester, Nitrile, wie Acetonitril oder Propionitril, Amide, wie Dimethylformamid, Dimethylacetamid, N-Methylpyrrolidon oder Hexamethylphosphorsäuretriamid. Verwendet man als Reaktionspartner Alkylierungsmittel der Formel (VI) in flüssiger Form, so ist es auch möglich, diese in entsprechendem Überschuß gleichzeitig als Verdünnungsmittel einzusetzen.

Als Reaktionshilfsmittel zur Durchführung des erfindungsgemäßen Verfahrens (c) kommen alle üblicherweise verwendbaren anorganischen und organischen Basen infrage. Vorzugsweise verwendet man Alkalimetallhydride, -hydroxide, -amide, -carbonate oder -hydrogencarbonate, wie beispielsweise Natriumhydrid, Natriumamid, Natriumhydroxid, Natriumcarbonat oder Natriumhydrogencarbonat, oder auch tertiäre Amine, wie beispielsweise Triethylamin, N,N-Dimethylanilin, Pyridin, 4-(N,N-Dimethylamino)-pyridin, Diazabicyclooctan (DABCO), Diazabicyclononen (DBN) oder Diazabicycloundecen (DBU).

Die Reaktionstemperaturen können bei der Durchführung des erfindungsgemäßen Verfahrens (c) in einem größeren Bereich variiert werden, Im allgemeinen arbeitet man zwischen -20 ° C und +150 ° C, vorzugsweise zwischen 0 ° C und +100 ° C.

Zur Durchführung des erfindungsgemäßen Verfahrens (c) setzt man pro Mol an Alkylidenstickstoffheterocyclus der Formel (Ia) im allgemeinen 1.0 bis 20.0 Mol, vorzugsweise 1.0 bis 15.0 Mol an Alkylierungsmittel der Formel (VI) und gegebenenfalls 1.0 bis 3.0 Mol, vorzugsweise 1.0 bis 2.0 Mol an Reaktionshilfsmittel ein.

Die Reaktionsdurchführung, Aufarbeitung und Isolierung der Reaktionsprodukte erfolgt nach allgemein üblichen Methoden (vergleiche auch die Herstellungsbeispiele).

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea, Trifolium, Ranunculus, Taraxacum.
Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.
Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.
Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso Können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht- und Hopfenanlagen, auf Zier- und Sportrasen und Weideflächen und zur selektiven Unkrautbekämpfung in einjährigen Kulturen eingesetzt werden.

Dabei lassen sich die erfindungsgemäßen Wirkstoffe mit besonders gutem Erfolg zur selektiven Bekämpfung von mono- und dikotylen Unkräutern in mono- und dikotylen Kulturen wie beispielsweise Weizen, Mais, Raps, Sonnenblumen oder Soja im Vorauflauf- oder im Nachauflaufverfahren einsetzen.

Daneben zeigen die erfindungsgemäßen Wirkstoffe in entsprechenden Aufwandmengen auch eine blattinsektizide und eine fungizide Wirksamkeit beispielsweise gegen den Erreger der Reisfleckenkrankheit (Pyricularia oryzae).

Die Wirkstoffe Können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen infrage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.
Als feste Trägerstoffe kommen infrage:
z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen infrage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen infrage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen infrage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide wie z.B. 1-Amino-6-ethylthio-3-(2,2-dimethylpropyl)-1,3,5-triazin-2,4(1H,3H)-dion (AMETHYDIONE) oder N-(2-Benzthiazolyl)-N,N'-dimethyl-harnstoff (METABENZTHIAZURON) zur Unkrautbekämpfung in Getreide; 4-Amino-3-methyl-6-phenyl-1,2,4-triazin-5-(4H)-on (METAMITRON) zur Unkrautbekämpfung in Zuckerrüben und 4-Amino-6-(1,1-dimethylethyl)-3-methylthio-1,2,4-triazin-5(4H)-on (METRIBUZIN) zur Unkrautbekämpfung in Sojabohnen, infrage. Auch Mischungen mit 2,4-Dichlorphenoxyessigsäure (2,4-D); 4-(2,4-Dichlorphenoxy)-buttersäure (2,4-DB); 2,4-Dichlorphenoxypropionsäure (2,4-DP); 5-(2-Chlor-4-trifluormethyl-phenoxy)-2-nitro-benzoesäure (ACIFLUORFEN); Chloressigsäure-N-(methoxymethyl)-2,6-diethylanilid (ALACHLOR); Methyl-6,6-dimethyl-2,4-dioxo-3-[1-(2-propenyloxyamino)-butyliden]-cyclohexancarbonsäure (ALLOXYDIM); 4-Amino-benzolsulfonyl-methylcarbamat (ASULAM); 2-Chlor-4-ethylamino-6-isopropylamino-1,3,5-triazin (ATRAZIN); 2-[[[[(4,6-Dimethoxypyrimidin-2-yl)-amino]-carbonyl]-amino]-sulfonyl]-methyl]-benzoesäuremethylester (BENSULFURON); 3-Isopropyl-2,1,3-benzothiadiazin-4-on-2,2-dioxid (BENTAZON); Methyl-5-(2,4-dichlorphenoxy)-2-nitrobenzoat (BIFENOX); 3,5-Dibrom-4-hydroxy-benzonitril (BROMOXYNIL); N-(Butoxymethyl)-2-chlor-N-(2,6-diethylphenyl)-acetamid (BUTACHLOR); 5-Amino-4-chlor-2-phenyl-2,3-dihydro-3-oxypyridazin (CHLORIDAZON); Ethyl-2-{[(4-chlor-6-methoxy-2-pyrimidinyl)-aminocarbonyl]-aminosulfonyl)-benzoat (CHLORIMURON); N-(3-Chlorphenyl)-isopropylcarbamat (CHLORPROPHAM); 2-Chlor-N-{[(4-methoxy-6-methyl-1,3,5-triazin-2-yl)-amino]-carbonyl}-benzolsulfonamid (CHLORSULFURON); N,N-Dimethyl-N'-(3-chlor-4-methylphenyl)-harnstoff (CHLORTOLURON); exo-1-Methyl-4-(1-methylethyl)-2-(2-methylphenyl-methoxy)-7-oxabicyclo-(2,2,1)-heptan (CINMETHYLIN); 3,6-Dichlor-2-pyridincarbonsäure (CLOPYRALID); 2-Chlor-4-ethylamino-6-(3-cyanopropylamino)-1,3,5-triazin (CYANAZIN); N,S-Diethyl-N-cyclohexylthiolcarbamat (CYCLOATE); 2-[1-(Ethoximino)-butyl]-3-hydroxy-5-[tetrahydro-(2H)-thiopyran-3-yl]-2-cyclohexen-1-on (CYCLOXYDIM); 2-[4-(2,4-Dichlorphenoxy)-phenoxy]-propionsäure, deren Methyl- oder deren Ethylester (DICLOFOP); 2-[(2-Chlorphenyl)-methyl]-4,4-dimethylisoxazolidin-3-on (DIMETHAZONE); N,N-Di-n-propyl-thiocarbamidsäure-S-ethylester (EPTAME); 4-Amino-6-t-butyl-3-ethylthio-1,2,4-triazin-5(4H)-on (ETHIOZIN); 2-{4-[(6-Chlor-2-benzoxazolyl)-oxy]-phenoxy}-propansäure, deren Methyl- oder deren Ethylester (FENOXAPROP); 2-[4-(5-Trifluormethyl-2-pyridyloxy)-phenoxy]-propansäure oder deren Butylester (FLUAZIFOP); N,N-Dimethyl-N'-(3-trifluormethylphenyl)-harnstoff (FLUOMETURON); 1-Methyl-3-phenyl-5-(3-trifluormethylphenyl)-4-pyridon (FLURIDONE); [(4-Amino-3,5-dichlor-6-fluor-2-pyridinyl)-oxy]-essigsäure bzw. deren 1-Methylheptylester (FLUROXYPYR); 5-(2-Chlor-4-trifluormethyl-phenoxy)-N-methylsulfonyl-2-nitrobenzamid (FOMESAFEN); 2-{4-[(3-Chlor-5-(trifluormethyl)-2-pyridinyl)-oxy]-phenoxy}-propansäure bzw. deren Ethylester (HALOXYFOP); 3-Cyclohexyl-6-dimethylamino-1-methyl-1,3,5-triazin-2,4-dion (HEXAZINONE); Methyl-2-[4,5-dihydro-4-methyl-4-(1-methylethyl)-5-oxo-1H-imidazol-2-yl]-4(5)-methylbenzoat (IMAZAMETHABENZ); 2-[5-Methyl-5-(1-methylethyl)-4-oxo-2-imidazolin-2-yl]-3-chinolincarbonsäure (IMAZAQUIN); 2-[4,5-Dihydro-4-methyl-4-isopropyl-5-oxo-(1H)-imidazol-2-yl]-5-ethyl-pyridin-3-carbonsäure (IMAZETHAPYR); 3,5-Diiod-4-hydroxybenzonitril (IOXYNIL); N,N-Dimethyl-H'-(4-isopropylphenyl)-harnstoff (ISOPROTURON); (2-Ethoxy-1-methyl-2-oxo-ethyl)-5-[2-chlor-4-(trifluormethyl)-phenoxyl-2-nitrobenzoat (LACTOFEN); (2-Methyl-4-chlorphenoxy)-essigsäure (MCPA); (4-Chlor-2-me-thylphenoxy)-propionsäure (MCPP); N-Methyl-2-(1,3-benzthiazol-2-yloxy)-acetanilid (MEFENACET); 2-Chlor-N-(2,6-dimethylphenyl)-N-[-(1H)-pyrazol-1-yl-methyl]-acetamid (METAZACHLOR); 2-Ethyl-6-methyl-N-(1-methyl-2-methoxyethyl)-chloracetanilid (METOLACHLOR); 2-{[[((4-Methoxy-6-methyl-1,3,5-triazin-2-yl)-amino)-carbonyl]-amino]-sulfonyl}-benzoesäure oder deren Methylester (METSULFURON); 1-(3-Trifluormethyl-phenyl)-4-methylamino-5-chlor-6-pyridazon (NORFLURAZON); 4-(Di-n-propylamino)-3,5-dinitrobenzolsulfonamid (ORYZALIN); (2-Chlor-4-trifluormethylphenyl)-(3-ethoxy-4-nitro-phenyl)-ether (OXYFLUORFEN); N-(1-Ethylpropyl)-3,4-dimethyl-2,6-dinitroanilin (PENDIMETHALIN); 3-(Ethoxycarbonylaminophenyl)-N-(3'-methylphenyl)-carbamat (PHENMEDIPHAM); 2-Chlor-N-isopropylacetanilid (PROPACHLOR); Isopropyl-N-phenyl-carbamat (PROPHAM); 0-(6-Chlor-3-phenyl-pyridazin-4-yl)-S-octylthiocarbonat (PYRIDATE); 2-[4-(6-Chlor-chinoxalin-2-yl-oxy)-phenoxy]-propionsäure-ethylester (QUIZALOFOPETHYL); 2-[1-(Ethoxamino)-butyliden]-5-(2-ethylthiopropyl)-1,3-cyclohexadion (SETHOXYDIM); 2-Chlor-4,6-bis-(ethylamino)-1,3,5-triazin (SIMAZIN); 2,4-Bis-[N-ethylamino]-6-methylthio-1,3,5-triazin (SIMETRYNE); 4-Ethylamino-2-t-butylamino-6-methylthio-s-triazin (TERBUTRYNE); 3-[[[[(4-Methoxy-6-methyl-1,3,5-triazin-2-yl)-amino]-carbonyl]-amino]-sulfonyl]-thiophen-2-carbonsäure-methylester (THIAMETURON); S-[(4-Chlorphenyl)-methyl]-N,N-diethyl-thiocarbamat (THIOBENCARB); N,N-Diisopropyl-S-(2,3,3-trichlorallyl)-thiolcarbamat (TRIALLATE); 2,6-Dinitro-4-trifluormethyl-N,N-dipropylanilin (TRIFLURALIN) sind möglich. Einige Mischungen zeigen überraschenderweise auch synergistische Wirkung.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden.

Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 0,001 und 10 kg Wirkstoff pro Hektar Bodenfläche, vorzugsweise zwischen 0,005 und 5 kg pro ha.

Die Herstellung und die Verwendung der erfindungsgemäßen Wirkstoffe geht aus den nachfolgenden Beispielen hervor.

Herstellungsbeispiele:

Beispiel 1:

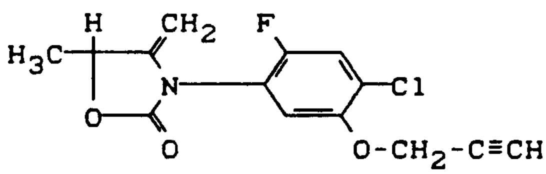

(Verfahren a)

Zu einer Lösung von 4.44 g (0.015 Mol) H-(4-Chlor-2-fluor-5-propargyloxyphenyl)-carbaminsäure-3-butinylester in 25 ml Dichlormethan gibt man 0.31 g (0.0045 Mol) Natriumethylat und rührt 24 Stunden bei Raumtemperatur. Zur Aufarbeitung versetzt man die Reaktionsmischung mit Wasser, trennt die organische Phase ab, trocknet über Natriumsulfat und entfernt das Lösungsmittel im Vakuum.

Man erhält 4.05 g (91 % der Theorie) an 3-(4-Chlor-2-fluor-5-propargyloxyphenyl)-5-methyl-4-methyliden-1,3-oxazolidin-2-on vom Schmelzpunkt 128 ° C.

Beispiel IV-1:

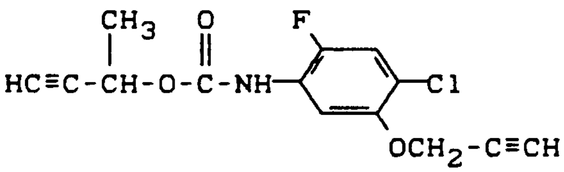

(Verfahren a)

Zu 5.64 g (0.025 Mol) 4-Chlor-2-fluor-5-propargyloxyphenylisocyanat gibt man bei Raumtemperatur tropfenweise unter Rühren eine Lösung von 1.93 g (0.0276 Mol) 3-Butinol in 5 ml Pyridin und erhitzt nach beendeter Zugabe für 4 Stunden auf 100 ° C. Zur Aufarbeitung wird die Reaktionsmischung abgekühlt, in ein Gemisch aus 5 ml konzentrierter Salzsäure und 40 ml Wasser gegeben, mit Dichlormethan extrahiert, über Natriumsulfat getrocknet und im Vakuum eingeengt.

Man erhält 6.11 g (83 % der Theorie) an N-(4-Chlor-2-fluor-5-propargyloxyphenyl)-carbaminsäure-3-butinylester vom Schmelzpunkt 45 ° C bis 50 ° C.

Beispiel 2:

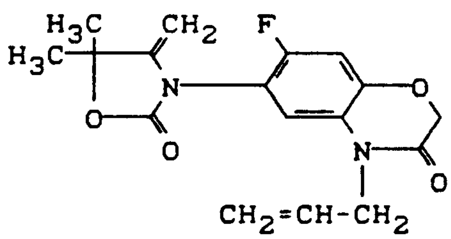

(Verfahren a)

Zu einer Lösung von 3.32 g (0.01 Mol) N-(7-Fluor-3,4-dihydro-3-oxo-4-allyl-2H-1,4-benzoxazin-6-yl)-carbaminsäure 3-methylbut-3-yr-2-ylester in 17 ml Toluol gibt man 0.10 g (0.0015 Mol) Natriummethylat. Man rührt noch eine Stunde bei Raumtemperatur nach, fügt Wasser hinzu und trennt die Phasen. Nach Waschen der Methylenchloridschicht mit gesättigter Kochsalzlösung trocknet man über Natriumsulfat, filtriert und engt ein. Man erhält 2.98 g (89.8 % der Theorie) 3-(7-Fluor-3,4-dihydro-3-oxo-4-allyl-2H-1,4-benzoxazin-6-yl)-5,5-dimethyl-4-methyliden-1,3-oxazolidin-2-on vom Schmelzpunkt 161 ° C bis 166 ° C.

Beispiel IV-2:

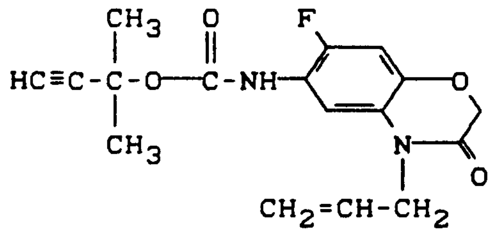

(Verfahren a)

Eine Lösung von 4.64 g (0.055 Mol) 3-Methyl-3-butinol in 10 ml Pyridin tropft man bei Raumtemperatur zu 12.41 g (0.05 Mol) 7-Fluor-3,4-dihydro-6-isocyanato-3-oxo-4-allyl-2H-1,4-benzoxazin und erhitzt nach beendeter Zugabe noch 1 Stunde auf 100 °C. Zur Aufarbeitung wird die Reaktionsmischung abgekühlt, in ein Gemisch aus 10 ml konzentrierter Salzsäure und 80 ml Wasser gegeben, mit Dichlormethan extrahiert, über Natriumsulfat getrocknet und im Vakuum eingeengt. Nach Säulenchromatografie über Kieselgel erhält man 8.89 g (53.6 % der Theorie) an N-(7-Fluor-3,4-dihydro-3-oxo-4-allyl-2H-1,4-benzoxazin-6-yl)-carbaminsäure(3-methyl-3-butinylester) vom Schmelzpunkt 107 °C bis 112 °C.

Beispiel 3:

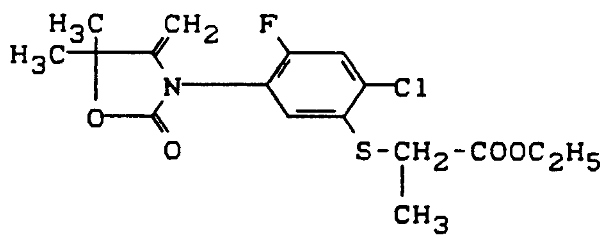

(Verfahren a / Eintopfverfahren)

Zu einer Lösung von 3.72 g (0.044 Mol) 3-Methyl-3-butinol in 10 ml Pyridin gibt man bei Raumtemperatur tropfenweise unter Rühren 12.38 g (0.041 Mol) 4-Chlor-2-fluor-5-[(1-ethoxycarbonyl)ethylthio]-phenylisocyanat und rührt nach beendeter Zugabe 7 Stunden bei 100 °C. Zur Aufarbeitung gibt man die abgekühlte Reaktionsmischung in vierprozentige wässrige Salzsäure, extrahiert mit Dichlormethan, trocknet über Natriumsulfat, engt im Vakuum ein und reinigt den Rückstand durch Chromatographie an Kieselgel.

Man erhält 1.48 g (9.4 % der Theorie) an 3-[4-Chlor-2-fluor-5-[(1-ethoxycarbonyl)-ethylthio]-phenyl]-5,5-dimethyl-4-methyliden-1,3-oxazolidin-2-on als Öl.

$^1$H-NMR ($CDCl_3$ / Tetramethylsilan):

$\delta$ = 7.58 (d; 1 H; J = 8 Hz); 7.37 (d; 1 H; J = 9 Hz); 4.13 (d; 1 H; J = 1 Hz); 4.11 (q; 2 H; J = 8 Hz); 3.99 (d; 1 H; J = 1 Hz); 3.88 (q; 1 H; J = 8 Hz); 1.65 (s; 6 H); 1.53 (d; 3 H; J = 8 Hz); 1.18 (t; 3 H; J = 8 Hz) ppm.

Beispiel 4:

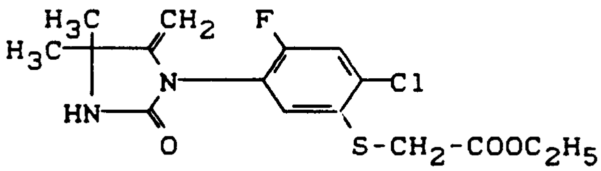

(Verfahren a)

Zu einer Suspension von 6.98 g (0.025 Mol) 1-(3-Methylbut-1-yr-3-yl)-3-(4-chlor-2-fluor-5-ethoxycarbonylmethylthiophenyl)harnstoff in 40 ml Toluol gibt man 0.68 g (0.01 Mol) Natriummethylat und erhitzt anschließend 7 Stunden bei 100 ° C. Nach Einengen trennt man den Rückstand durch Säulenchromatografie über Kieselgel auf.

Man erhält 2.03 g (29.0 % der Theorie) 3-(4-Chlor-2-fluor-5-ethoxycarbonylmethylthiophenyl)-5,5-dimethyl-4-methylidenimidazolidin-2-on als Öl.

$^1$H-NMR ($CDCl_3$ / Tetramethylsilan)

δ = 7.46 (d; 1 H; J = 8 Hz); 7.32 (d; 1 H; J = 9 Hz); 5.74 (bs; 1 H); 4.16 (q; 2 H; J = 8 Hz); 4.04 (d; 1 H; J = 1 Hz); 3.88 (s; 1 H); 3.65 (s; 2 H); 1.48 (s; 6 H); 1.24 (t, 3 H, J = 8 Hz) ppm.

Beispiel IV-3:

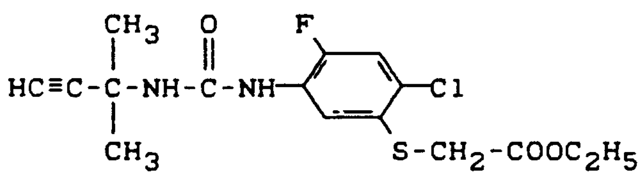

(Verfahren a)

Zu 11.6 g (0.04 Mol) 4-Chlor-2-fluor-5-ethoxycarbonylmethylthio-phenylisocyanat in 10 ml Toluol gibt man bei Raumtemperatur tropfenweise unter Rühren eine Lösung von 3.66 g (0.044 Mol) 3,3-Dimethylpropargylamin in 10 ml Toluol und rührt anschließend eine Stunde bei Raumtemperatur.

Zur Aufarbeitung werden Lösungsmittel und flüchtige Bestandteile durch Destillation im Vakuum entfernt.

Man erhält 14.91 g (100 % der Theorie) an N-(3-Methyl-3-butinyl)-N'-(4-Chlor-2-fluor-5-ethoxycarbonylmethylthiophenyl)harnstoff als Öl.

$^1$H-NMR ($CDCl_3$ / Tetramethylsilan):

δ = 8.4 (d; 1 H; J = 8 Hz); 7.28 (s, 1H); 7.07 (d; 1 H; J = 9 Hz); 5.74 (s; 1 H); 4.19 (q, 2 H; J = 8 Hz); 3.62 (s; 2 H); 2.39 (s; 1 H); 1.62 (s; 6 H); 1.28 (t; 3 H; J = 8 Hz) ppm.

Beispiel 5:

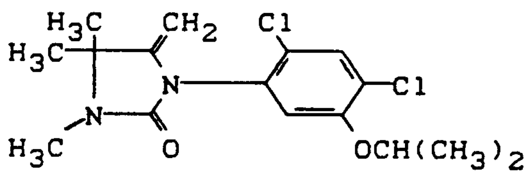

(Verfahren c)

Ein Gemisch aus 3.20 g (0.01 Mol) 3-(2,4-Dichlor-5-isopropoxyphenyl)-5,5-dimethyl-4-methylidenimidazolidin-2-on, 2.76 g (0.02 Mol) Kaliumcarbonat und 60 ml Acetonitril erhitzt man 2 Stunden auf Rückflußtemperatur. Nach Abkühlen auf Raumtemperatur fügt man 35.5 g (0.25 Mol) Methyliodid hinzu und erhitzt weitere 49 Stunden auf Rückflußtemperatur. Man engt ein, versetzt den Rückstand mit Wasser und Methylenchlorid und trennt die Methylenchloridphase ab. Nach Trocknen über Natriumsulfat, Abfiltrieren und Einengen erhält man 3,42 g (99.7 % der Theorie) 3-(2,4-Dichlor-5-isopropoxyphenyl)-1,5,5-trimethyl-4-methylidenimidazolidin-2-on vom Schmelzpunkt 92° C bis 104° C.

In entsprechender Weise und gemäß den allgemeinen Angaben zur Herstellung erhält man die folgenden arylsubstituierten Alkylidenstickstoffheterocyclen der allgemeinen Formel (I) sowie die folgenden Alkinylcarbamate und -harnstoffe der allgemeinen Formel (IV):

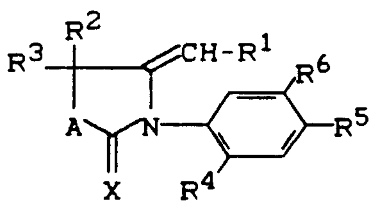

(I)

| Bsp.Nr. | $R^1$ | $R^2$ | $R^3$ | $R^4$ | $R^5$ | $R^6$ | A | X | physikalische Eigenschaften |
|---|---|---|---|---|---|---|---|---|---|
| 6 | H | H | $CH_3$ | F | Cl | $-S-CH_2-COOC_2H_5$ | O | O | $^1$H-NMR*): 7.48(d);7.35(d) 1.63(d);1.23(t) |
| 7 | H | H | $CH_3$ | F | $-O-CH_2-C-N-$ (C=O; N–$CH_2-CH=CH_2$)<br>$\|\| \quad \|$<br>$O \quad CH_2-CH=CH_2$ | | O | O | $^1$H-NMR*): 6.9(d);5.84(m) 4.0(s);1.63(d) |
| 8 | H | $CH_3$ | $CH_3$ | Cl | Cl | $-O-CH(CH_3)_2$ | O | O | Fp.:160° C |
| 9 | H | $CH_3$ | $CH_3$ | H | Cl | $-S-CH_2-COOCH_3$ | O | O | Fp.:88-92° C |
| 10 | H | $CH_3$ | $CH_3$ | F | $CH_3$ | $-O-CH(CH_3)_2$ | O | O | Fp.:136-138° C |
| 11 | H | $CH_3$ | $CH_3$ | F | Cl | $-S-CH_2-COOC_2H_5$ | O | O | Fp.:86-91° C |
| 12 | H | $CH_3$ | $CH_3$ | F | $-O-CH_2-C-N-$<br>$\|\| \quad \|$<br>$O \quad CH_2-C \equiv CH$ | | O | O | Fp.:134-138° C |
| 13 | H | $CH_3$ | $CH_3$ | Cl | Cl | $-O-CH(CH_3)_2$ | NH | O | Fp.:184-186° C |
| 14 | H | $CH_3$ | $CH_3$ | H | H | $CF_3$ | O | O | Fp.:85-92° C |

*) Die $^1$H-NMR-Spektren wurden in Deuterochloroform ($CDCl_3$) mit Tetramethylsilan (TMS) als innerem Standard aufgenommen. Angegeben ist die chemische Verschiebung als δ-Wert in ppm.

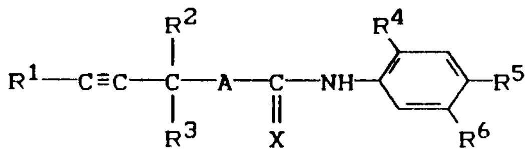

(IV)

| Bsp.Nr. | $R^1$ | $R^2$ | $R^3$ | $R^4$ | $R^5$ | $R^6$ | A | X | physikalische Eigenschaften |
|---|---|---|---|---|---|---|---|---|---|
| IV-4 | H | H | $CH_3$ | F | Cl | $-S-CH_2-COOC_2H_5$ | O | O | $^1$H-NMR*): 8.28(d);7.16(d) 1.58(d);1.26(d) |
| IV-5 | H | H | $CH_3$ | F | $-O-CH_2-C-N-$<br>‖ \|<br>$O\ CH_2-CH=CH_2$ | | O | O | $^1$H-NMR*): 7.85(d);6.77(d) 1.57(d) |
| IV-6 | H | $CH_3$ | $CH_3$ | Cl | Cl | $-O-CH(CH_3)_2$ | O | O | Fp.:77-81°C |
| IV-7 | H | $CH_3$ | $CH_3$ | H | Cl | $-S-CH_2-COOCH_3$ | O | O | Fp.:85-93°C |
| IV-8 | H | $CH_3$ | $CH_3$ | F | Cl | $-S-CH_2-COOC_2H_5$ | O | O | Fp.:78-85°C |
| IV-9 | H | $CH_3$ | $CH_3$ | H | $-O-CH_2-C-N-$<br>‖ \|<br>$O\ CH_2-C\equiv CH$ | | O | O | Fp.:151-153°C |

*) Die $^1$H-NMR-Spektren wurden in Deuterochloroform ($CDCl_3$) mit Tetramethylsilan (TMS) als innerem Standard aufgenommen. Angegeben ist die chemische Verschiebung als δ-Wert in ppm.

| Bsp.Nr. | $R^1$ | $R^2$ | $R^3$ | $R^4$ | $R^5$ | $R^6$ | A | X | physikalische Eigenschaften |
|---|---|---|---|---|---|---|---|---|---|
| IV-10 | H | $CH_3$ | $CH_3$ | F | $CH_3$ | $-O-CH(CH_3)_2$ | O | O | ¹H-NMR*): 6.82(d);2.13(s) 1.75(s);1.3(d) |
| IV-11 | H | $-(CH_2)_5-$ | | F | $-O-CH_2-C-N-$<br>‖ \|<br>$O\ CH_2-CH=CH_2$ | | O | O | Fp.:142-148° C |
| IV-12 | H | $CH_3$ | $CH_3$ | H | H | $CF_3$ | O | O | Fp.:79-82° C |
| IV-13 | H | $CH_3$ | $CH_3$ | Cl | Cl | $-O-CH(CH_3)_2$ | O | O | Fp.:177-179° C |

*) Die ¹H-NMR-Spektren wurden in Deuterochloroform ($CDCl_3$) mit Tetramethylsilan (TMS) als innerem Standard aufgenommen. Angegeben ist die chemische Verschiebung als δ-Wert in ppm.

Anwendungsbeispiele:

In den folgenden Anwendungsbeispielen wurde die nachstehend aufgeführte Verbindung als Vergleichsubstanz eingesetzt:

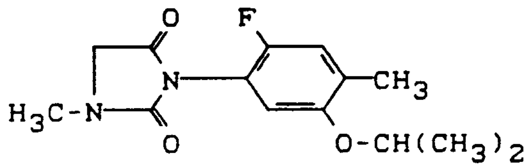

(A)

1-(2-Fluor-5-isopropoxy-4-methylphenyl)-3-methyl-imidazolidin-2,5-dion (bekannt aus EP 363 585)

Beispiel A

Pre-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:

0 % = keine Wirkung (wie unbehandelte Kontrolle)
100 % = totale Vernichtung

Eine deutliche Überlegenheit in der Wirksamkeit ebenso wie in der Nutzpflanzenselektivität gegenüber dem Stand der Technik zeigen in diesem Test z.B. die Verbindungen gemäß folgender Herstellungsbeispiele: 1 und 7.

Beispiel B

Post-emergence-Test

Lösungsmittel: 5 Gewichtsteile Aceton
Emulgator: 1 Gewichtsteil Alkylarylpolyglykolether sowie 0,1 % Renex 36

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben, so, daß die jeweils gewünschten Wirkstoffmengen pro Flächeneinheit ausgebracht werden. Die Konzentration der Spritzbrühe wird so gewählt, daß in 1000 l Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrolle.

Es bedeuten:

0 % = keine Wirkung (wie unbehandelte Kontrolle)
100 % = totale Vernichtung

Eine deutliche Überlegenheit in der Wirksamkeit ebenso wie in der Nutzpflanzenselektivität gegenüber dem Stand der Technik zeigen in diesem Test z.B. die Verbindungen gemäß folgender Herstellungsbeispiele: 1, 6, 7 und 11.

**Patentansprüche**

**1.** Arylsubstituierte Alkylidenstickstoffheterocyclen der allgemeinen Formel (I),

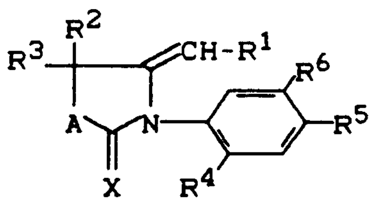

(I)

in welcher

$R^1$ für Wasserstoff, Halogen, Alkyl oder Aryl steht,

$R^2$ und $R^3$ unabhängig voneinander jeweils für Wasserstoff, Alkyl oder Cycloalkyl stehen oder gemeinsam für einen zweifach verknüpften Alkandiylrest stehen,

$R^4$ für Wasserstoff oder Halogen steht,

$R^5$ für Wasserstoff, Halogen, Cyano oder Alkyl steht und

$R^6$ für Halogenalkyl oder für einen Rest $-X^1-R^7$ steht oder

$R^5$ und $R^6$ gemeinsam für einen zweifach verknüpften Rest der Formel

$$-X^1-(A^1)_m-(\underset{\underset{O}{\|}}{C})_n-X^2-\text{stehen,}$$

A für Sauerstoff oder für einen Rest $>N-R^8$ steht und

X für Sauerstoff oder Schwefel steht, wobei

$R^7$ für Wasserstoff, Alkyl, Alkenyl, Alkinyl, Cyanalkyl, Alkoxyalkyl, Alkoxyalkoxyalkyl, Alkoxyalkoxyalkoxyalkyl, Alkoxycarbonylalkyl, Heterocyclylalkyl oder Heterocyclyl steht,

$R^8$ für Wasserstoff, Alkyl oder Halogenalkyl steht,

$A^1$ für zweifach verknüpftes Alkandiyl steht,

$X^1$ für Sauerstoff oder Schwefel steht,

$X^2$ für Sauerstoff, Schwefel oder $>N-R^7$ steht,

n für eine Zahl 0 oder 1 steht und

m für eino Zahl 0 oder 1 steht.

**2.** Arylsubstituierte Alkylidenstickstoffheterocyclen der allgemeinen Formel (I) gemäß Anspruch 1, dadurch gekennzeichnet, daß darin

$R^1$ für Wasserstoff, Fluor, Chlor, Brom, für geradkettiges oder verzweigtes Alkyl mit 1 bis 8 Kohlenstoffatomen oder für Phenyl steht,

$R^2$ und $R^3$ entweder unabhängig voneinander jeweils für Wasserstoff, für geradkettiges oder verzweigtes Alkyl mit 1 bis 8 Kohlenstoffatomen oder für Cycloalkyl mit 3 bis 7 Kohlenstoffatomen stehen oder gemeinsam für einen zweifach verknüpften geradkettigen oder verzweigten Alkandiylrest mit 2 bis 10 Kohlenstoffatomen stehen,

$R^4$ für Wasserstoff, Fluor, Chlor oder Brom steht,

$R^5$ für Wasserstoff, Fluor, Chlor, Brom, Cyano oder für geradkettiges oder verzweigtes Alkyl mit 1 bis 8 Kohlenstoffatomen steht und

$R^6$ für geradkettiges oder verzweigtes Halogenalkyl mit 1 bis 6 Kohlenstoffatomen und 1 bis 13 gleichen oder verschiedenen Halogenatomen oder für einen Rest $-X^1-R^7$ steht oder

$R^5$ und $R^6$ gemeinsam für einen zweifach verknüpften Rest der Formel

$$-X^1-(A^1)_m-(\underset{\underset{O}{\|}}{C})_n-X^2-$$

stehen,

A für Sauerstoff oder für einen Rest >N-$R^8$ steht und

X für Sauerstoff oder Schwefel steht, wobei

$R^7$ für Wasserstoff, für jeweils geradkettiges oder verzweigtes Alkyl mit 1 bis 12 Kohlenstoffatomen, Alkenyl mit 2 bis 12 Kohlenstoffatomen, Alkinyl mit 2 bis 12 Kohlenstoffatomen, Cyanalkyl mit 2 bis 12 Kohlenstoffatomen, Alkoxyalkyl, Alkoxyalkoxyalkyl, Alkoxyalkoxyalkoxyalkyl oder Alkoxycarbonylalkyl mit jeweils 1 bis 8 Kohlenstoffatomen in den einzelnen Alkoxy- und Alkylteilen, für Heterocyclylalkyl mit 1 bis 4 Kohlenstoffatomen im geradkettigen oder verzweigten Alkylteil oder für Heterocyclyl steht, wobei als Heterocyclylrest jeweils ein 3- bis 7-gliedriger gegebenenfalls auch benzannellierter oder mehrkernig kondensierter, gesättigter oder ungesättigter Heterocyclus mit 1 bis 5 Heteroatomen - insbesondere Stickstoff, Sauerstoff und/oder Schwefel- und 2 bis 10 Kohlenstoffatomen infrage kommt,

$R^8$ für Wasserstoff, für geradkettiges oder verzweigtes Alkyl mit 1 bis 8 Kohlenstoffatomen oder für geradkettiges oder verzweigtes Halogenalkyl mit 1 bis 4 Kohlenstoffatomen und 1 bis 9 gleichen oder verschiedenen Halogenatomen steht,

$A^1$ für geradkettiges oder verzweigtes zweifach verknüpftes Alkandiyl mit 1 bis 6 Kohlenstoffatomen steht,

$X^1$ für Sauerstoff oder Schwefel steht,

$X^2$ für Sauerstoff, Schwefel oder >N-$R^7$ steht,

n für eine Zahl 0 oder 1 steht und

m für eine Zahl 0 oder 1 steht.

**3.** Arylsubstituierte Alkylidenstickstoffheterocyclen der allgemeinen Formel (I) gemäß Anspruch 1, dadurch gekennzeichnet, daß darin

$R^1$ für Wasserstoff, Fluor, Chlor, Brom, Methyl, Ethyl, n- oder i-Propyl, n-, i-, s- oder t-Butyl oder für Phenyl steht,

$R^2$ und $R^3$ entweder unabhängig voneinander jeweils für Wasserstoff, Methyl, Ethyl, n- oder i-Propyl, n-, i-, s- oder t-Butyl, für Cyclopropyl, Cyclopentyl oder Cyclohexyl stehen oder gemeinsam für einen jeweils zweifach verknüpften 1,2-Ethandiylrest, 1,3-Propandiylrest, 1,4-Butandiylrest oder 1,5-Pentandiylrest stehen,

$R^4$ für Wasserstoff, Fluor oder Chlor steht,

$R^5$ für Wasserstoff, Fluor, Chlor, Cyano, Methyl, Ethyl, n- oder i-Propyl, n-, i-, s- oder t-Butyl steht und

$R^6$ für geradkettiges oder verzweigtes Halogenalkyl mit 1 bis 4 Kohlenstoffatomen und 1 bis 9 gleichen oder verschiedenen Halogenatomen oder für einen Rest -$X^1$-$R^7$ steht oder

$R^5$ und $R^6$ gemeinsam für einen zweifach verknüpften Rest der Formel

$$-X^1-(A^1)_m-(\underset{\underset{O}{\|}}{C})_n-X^2-$$

stehen,

A für Sauerstoff oder für einen Rest >N-$R^8$ steht und

X für Sauerstoff oder Schwefel steht, wobei

$R^7$ für Wasserstoff, Methyl, Ethyl, n- oder i-Propyl, n-, i-, s oder t-Butyl, für Allyl, n-oder i-Butenyl, n- oder i-Pentenyl, n- oder i-Hexenyl, Propargyl, n- oder i-Butinyl, n-oder i-Pentinyl, n- oder i-Hexinyl, für Cyanmethyl, Cyanethyl, Cyanpropyl, für Methoxymethyl, Methoxyethyl, Methoxypropyl, Ethoxymethyl, Ethoxyethyl, Ethoxypropyl, Propoxymethyl, Propoxyethyl, Propoxypropyl, für Methoxyethoxyethyl, für Ethoxyethoxyethyl, für Propoxyethoxyethyl, für Methoxycarbonylmethyl, Ethoxycarbonylmethyl, Propoxycarbonylmethyl, Methoxycarbonylethyl, Ethoxycarbonylethyl, Propoxycarbonylethyl, Methoxycarbonylpropyl, Ethoxycarbonylpropyl, Propoxycarbonylpropyl, für Heterocyclylmethyl, Heterocyclylethyl oder Heterocyclyl steht, wobei als Heterocyclylrest ein fünfgliedriger oder 6-gliedriger, gesättigter oder ungesättigter Heterocyclus

mit 1 bis 3 Heteroatomen, insbesondere Stickstoff, Sauerstoff und/oder Schwefel und 2 bis 5 Kohlenstoffatomen infrage kommt,

$R^8$ für Wasserstoff, Methyl, Ethyl, n- oder i-Propyl, n-, i-, s- oder t-Butyl oder für Halogenalkyl mit 1 bis 2 Kohlenstoffatomen und 1 bis 5 gleichen oder verschiedenen Halogenatomen steht,

$A^1$ für geradkettiges oder verzweigtes, zweifach verknüpftes Alkandiyl mit 1 bis 4 Kohlenstoffatomen steht,

$X^1$ für Sauerstoff oder Schwefel steht,

$X^2$ für Sauerstoff, Schwefel oder $>N-R^7$ steht,

n für eine Zahl 0 oder 1 steht und

m für eine Zahl 0 oder 1 steht.

**4.** Arylsubstituierte Alkylidenstickstoffheterocyclen der allgemeinen Formel (I) gemäß Anspruch 1, dadurch gekennzeichnet, daß darin

$R^1$ für Wasserstoff, Chlor, Methyl, Ethyl, n- oder i-Propyl oder Phenyl steht,

$R^2$ und $R^3$ entweder unabhängig voneinander jeweils für Wasserstoff, Methyl, Ethyl, n- oder i-Propyl, oder Cyclopropyl, Cyclopentyl oder Cyclohexyl stehen, oder gemeinsam für einen 1,2-Ethandiylrest, einen 1,3-Propandiylrest, einen 1,4-Butandiylrest oder einen 1,5-Pentandiylrest stehen,

$R^4$ für Wasserstoff, Fluor oder Chlor steht

$R^5$ für Wasserstoff, Chlor, Brom, Cyano oder Methyl steht und

$R^6$ für Trifluormethyl oder für einen Rest $-X^1-R^7$ steht oder

$R^5$ und $R^6$ gemeinsam für einen zweifach verknüpften Rest der Formel

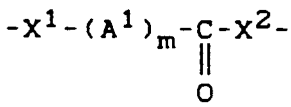

stehen,

A für Sauerstoff oder für einen Rest $>N-R^8$ steht und

X für Sauerstoff oder Schwefel steht, wobei

$R^7$ für Wasserstoff, Methyl, Ethyl, n- oder i-Propyl, Allyl, n- oder i-Butenyl, Propargyl, n- oder i-Butinyl, Cyanmethyl, Ethoxyethyl, Ethoxyethoxyethyl, Methoxycarbonylmethyl, Ethoxycarbonylmethyl, Propoxycarbonylmethyl, Methoxycarbonylethyl, Ethoxycarbonylethyl, Propoxycarbonylethyl, Methoxycarbonylpropyl, Ethoxycarbonylpropyl, Propoxycarbonylpropyl, für Pyridyl, Pyridylmethyl oder Pyridylethyl steht,

$R^8$ für Wasserstoff, Methyl, Ethyl, n- oder i-Propyl, Fluormethyl, Chlormethyl, Brommethyl, Difluormethyl oder Trifluormethyl steht,

$A^1$ für einen der Reste $-CH_2-$,

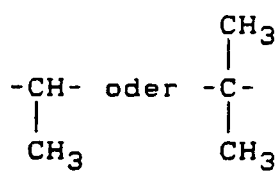
oder

steht,

$X^1$ für Sauerstoff oder Schwefel steht,

$X^2$ für Sauerstoff, Schwefel oder $>N-R^7$ steht und

m für eine Zahl 0 oder 1 steht.

**5.** Verfahren zur Herstellung von arylsubstituierten Alkylidenstickstoffheterocyclen der allgemeinen Formel (I),

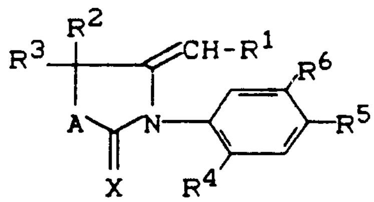

(I)

in welcher

- $R^1$ für Wasserstoff, Halogen, Alkyl oder Aryl steht,
- $R^2$ und $R^3$ unabhängig voneinander jeweils für Wasserstoff, Alkyl oder Cycloalkyl stehen oder gemeinsam für einen zweifach verknüpften Alkandiylrest stehen,
- $R^4$ für Wasserstoff oder Halogen steht,
- $R^5$ für Wasserstoff, Halogen, Cyano oder Alkyl steht und
- $R^6$ für Halogenalkyl oder für einen Rest $-X^1-R^7$ steht oder
- $R^5$ und $R^6$ gemeinsam für einen zweifach verknüpften Rest der Formel

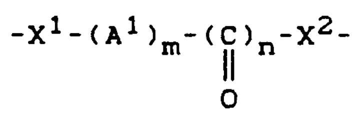

stehen,

- A für Sauerstoff oder für einen Rest $>N-R^8$ steht und
- X für Sauerstoff oder Schwefel steht, wobei
- $R^7$ für Wasserstoff, Alkyl, Alkenyl, Alkinyl, Cyanalkyl, Alkoxyalkyl, Alkoxyalkoxyalkyl, Alkoxyalkoxyalkoxyalkyl, Alkoxycarbonylalkyl, Heterocyclylalkyl oder Heterocyclyl steht,
- $R^8$ für Wasserstoff, Alkyl oder Halogenalkyl steht,
- $A^1$ für zweifach verknüpftes Alkandiyl steht,
- $X^1$ für Sauerstoff oder Schwefel steht,
- $X^2$ für Sauerstoff, Schwefel oder $>N-R^7$ steht,
- n für eine Zahl 0 oder 1 steht und
- m für eine Zahl 0 oder 1 steht,

dadurch gekennzeichnet, daß man

(a) Alkinylverbindungen der Formel (II),

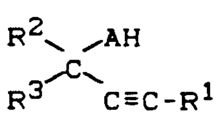

(II)

in welcher

$R^1$, $R^2$, $R^3$ und A die oben angegebene Bedeutung haben,

mit Iso(thio)cyanaten der Formel (III),

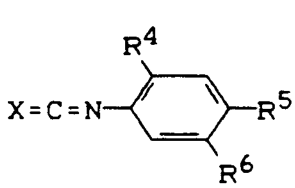

(III)

in welcher

$R^4$, $R^5$, $R^6$ und X die oben angegebene Bedeutung haben,

gegebenenfalls in Gegenwart eines Verdünnungsmittels und gegebenenfalls in Gegenwart eines Reaktionshilfsmittels umsetzt und anschließend die so erhältlichen Alkinylcarbamate (oder -harnstoffe) der Formel (IV),

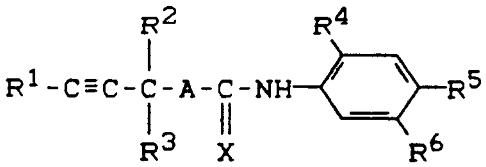

(IV)

in welcher

$R^1$, $R^2$, $R^3$, $R^4$, $R^5$, $R^6$, A und X die oben angegebene Bedeutung haben,

mit organischen Basen gegebenenfalls in Gegenwart eines Verdünnungsmittels cyclisiert;

oder daß man

(b) Alkinylverbindungen der Formel (IIa),

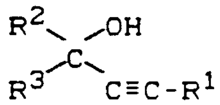

(IIa)

in welcher

$R^1$, $R^2$ und $R^3$ die oben angegebene Bedeutung haben,

mit Anilinderivaten der Formel (V),

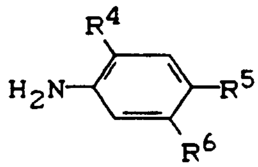

(V)

in welcher

$R^4$, $R^5$ und $R^6$ die oben angegebene Bedeutung haben,

in Gegenwart von Kohlendioxid sowie gegebenenfalls in Gegenwart eines Verdünnungsmittels und gegebenenfalls in Gegenwart eines Reaktionshilfsmittels umsetzt;

oder daß man

(c) die mit Hilfe des erfindungsgemäßen Verfahrens (a) erhältlichen arylsubstituierten Alkylidenstickstoffheterocyclen der Formel (Ia),

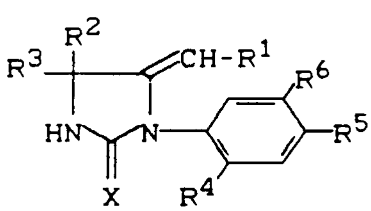

(Ia)

in welcher

$R^1$, $R^2$, $R^3$, $R^4$, $R^5$, $R^6$ und X die oben angegebene Bedeutung haben,
mit Alkylierungsmitteln der Formel (VI),

$R^{8-1}$-E (VI)

in welcher
$R^{8-1}$ für Alkyl oder Halogenalkyl steht und
E für eine elektronenanziehende Abgangsgruppe steht,
gegebenenfalls in Gegenwart eines Verdünnungsmittels und gegebenenfalls in Gegenwart eines Reaktionshilfsmittels umsetzt.

6. Herbizide Mittel, gekennzeichnet durch einen Gehalt an mindestens einem arylsubstituierten Alkylidenstickstoffheterocyclus der Formel (I) gemäß den Ansprüchen 1 bis 5.

7. Verfahren zur Bekämpfung von Unkräutern, dadurch gekennzeichnet, daß man arylsubstituierte Alkylidenstickstoffheterocyclen der Formel (I) gemäß den Ansprüchen 1 bis 5 auf die Unkräutern und/oder ihren Lebensraum einwirken läßt.

8. Verwendung der arylsubstituierten Alkylidenstickstoffheterocyclen der Formel (I) gemäß den Ansprüchen 1 bis 5 zur Bekämpfung von Unkräutern.

9. Arylsubstituierte Alkylidenstickstoffheterocyclen der Formel (Ia),

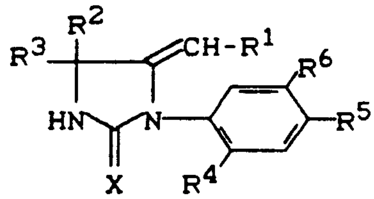

(Ia)

in welcher
$R^1$ für Wasserstoff, Halogen, Alkyl oder Aryl steht,
$R^2$ und $R^3$ unabhängig voneinander jeweils für Wasserstoff, Alkyl oder Cycloalkyl stehen oder gemeinsam für einen zweifach verknüpften Alkandiylrest stehen,
$R^4$ für Wasserstoff oder Halogen steht,
$R^5$ für Wasserstoff, Halogen, Cyano oder Alkyl steht und
$R^6$ für Halogenalkyl oder für einen Rest $-X^1-R^7$ steht oder
$R^5$ und $R^6$ gemeinsam für einen zweifach verknüpften Rest der Formel

$$-X^1-(A^1)_m-(\underset{\underset{O}{\|}}{C})_n-X^2-$$

stehen,
X für Sauerstoff oder Schwefel steht, wobei
$R^7$ für Wasserstoff, Alkyl, Alkenyl, Alkinyl, Cyanalkyl, Alkoxyalkyl, Alkoxyalkoxyalkyl, Alkoxyalkoxyalkoxyalkyl, Alkoxycarbonylalkyl, Heterocyclylalkyl oder Heterocyclyl steht,
$A^1$ für zweifach verknüpftes Alkandiyl steht,
$X^1$ für Sauerstoff oder Schwefel steht,
$X^2$ für Sauerstoff, Schwefel oder $>N-R^7$ steht,
n für eine Zahl 0 oder 1 steht und
m für eine Zahl 0 oder 1 steht.

10. Alkinylcarbamate (oder -harnstoffe) der Formel (IV),

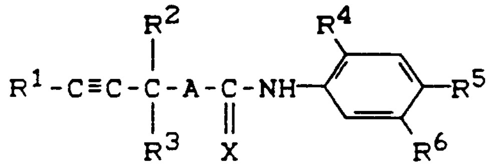

(IV)

in welcher

| | |
|---|---|
| $R^1$ | für Wasserstoff, Halogen, Alkyl oder Aryl steht, |
| $R^2$ und $R^3$ | unabhängig voneinander jeweils für Wasserstoff, Alkyl oder Cycloalkyl stehen oder gemeinsam für einen zweifach verknüpften Alkandiylrest stehen, |
| $R^4$ | für Wasserstoff oder Halogen steht, |
| $R^5$ | für Wasserstoff, Halogen, Cyano oder Alkyl steht und |
| $R^6$ | für Halogenalkyl oder für einen Rest $-X^1-R^7$ steht oder |
| $R^5$ und $R^6$ | gemeinsam für einen zweifach verknüpften Rest der Formel |

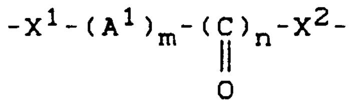

| | |
|---|---|
| | stehen, |
| A | für Sauerstoff oder für einen Rest $>N-R^8$ steht und |
| X | für Sauerstoff oder Schwefel steht, wobei |
| $R^7$ | für Wasserstoff, Alkyl, Alkenyl, Alkinyl, Cyanalkyl, Alkoxyalkyl, Alkoxyalkoxyalkyl, Alkoxyalkoxyalkoxyalkyl, Alkoxycarbonylalkyl, Heterocyclylalkyl oder Heterocyclyl steht, |
| $R^8$ | für Wasserstoff, Alkyl oder Halogenalkyl steht, |
| $A^1$ | für zweifach verknüpftes Alkandiyl steht, |
| $X^1$ | für Sauerstoff oder Schwefel steht, |
| $X^2$ | für Sauerstoff, Schwefel oder $>N-R^7$ steht, |
| n | für eine Zahl 0 oder 1 steht und |
| m | für eine Zahl 0 oder 1 steht. |

Europäisches Patentamt

# EUROPÄISCHER RECHERCHENBERICHT

Nummer der Anmeldung

EP 91 11 8315

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| A | DE-B-1 151 507 (BASF AKTIENGESELLSCHAFT)<br>* das ganze Dokument *<br>--- | 1,6,7 | C07D263/38<br>A01N43/76<br>C07D413/04<br>C07D233/70<br>A01N43/50<br>C07D263/46<br>C07C271/28<br>C07C275/28<br>C07C323/63<br>C07C275/34 |
| A | US-A-2 844 590 (M.D.CAMERON)<br>* Ansprüche *<br>--- | 1,5 | |
| A | EP-A-0 078 663 (SUMITOMO CHEMICAL COMPANY LIMITED)<br>* Ansprüche 1,2 *<br>--- | 10 | |
| A | JOURNAL OF AGRICULTURAL AND FOOD CHEMISTRY.<br>Bd. 27, Nr. 3, Mai 1979, WASHINGTON US<br>Seiten 537 - 543;<br>W.J.PYNE: 'Synthesis and herbicidal activity of N-alkyl-N-propargyl-N'-phenylureas and related compounds'<br>* das ganze Dokument *<br>--- | 10 | |
| P,A | EP-A-0 416 361 (BAYER AKTIENGESELLSCHAFT)<br>* Ansprüche *<br>----- | 1-10 | RECHERCHIERTE SACHGEBIETE (Int. Cl.5)<br>C07D<br>C07C<br>A01N |

Der vorliegende Recherchenbericht wurde für alle Patentansprüche erstellt

| Recherchenort | Abschlußdatum der Recherche | Prüfer |
|---|---|---|
| DEN HAAG | 27 JANUAR 1992 | HENRY J.C. |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)